(19) Europäisches Patentamt
European Patent Office
Office européen des brevets

(11) Publication number: 0 438 261 A2

# (12) EUROPEAN PATENT APPLICATION

(21) Application number: 91300266.3

(22) Date of filing: 15.01.91

(51) Int. Cl.[5]: C07D 487/04, C07D 495/04, C07D 491/048, C07D 239/70, A61K 31/505, // (C07D487/04, 239:00, 209:00), (C07D495/04, 333:00, 239:00), (C07D491/048, 307:00, 239:00)

The title of the invention has been amended (Guidelines for Examination in the EPO, A-III, 7.3).

(30) Priority: 16.01.90 JP 7962/90

(43) Date of publication of application: 24.07.91 Bulletin 91/30

(84) Designated Contracting States: AT BE CH DE DK ES FR GB GR IT LI LU NL SE

(71) Applicant: TAKEDA CHEMICAL INDUSTRIES, LTD.
3-6, Doshomachi 2-chome Chuo-ku
Osaka 541 (JP)

(72) Inventor: Akimoto, Hiroshi
4-25, Morikitamachi 6-chome, Higashinada-ku
Kobe, Hyogo 658 (JP)
Inventor: Ootsu, Koichiro
9-26, Todaiji 3-chome, Shimamoto-cho
Mishima-gun, Osaka 618 (JP)

(74) Representative: Laredo, Jack Joseph et al
Elkington and Fife Beacon House 113 Kingsway
London, WC2B 6PP (GB)

## (54) Condensed heterocyclic glutamic acid derivatives, their production and use.

(57) Novel compounds of the formula:

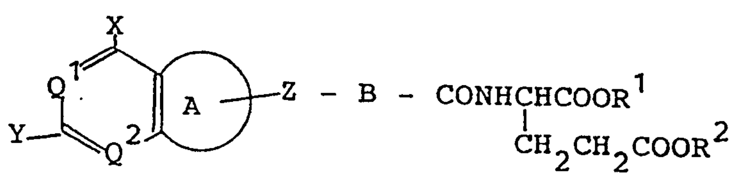

wherein the ring A is a 5-membered ring, provided that, in the case where the ring A is a pyrrole or pyrroline ring, (i) the N atom in the ring has no active proton or (ii) the ring bonds to Z at the -position of the ring;
B is a divalent cyclic or chain group which may be substituted; one of $Q^1$ and $Q^2$ is N, with the other being N or CH; Y is a hydrogen or halogen atom or a group which bonds to the bonding line with a carbon, nitrogen, oxygen or sulfur atom; X is an amino, hydroxyl or mercapto group;
Z is a straight-chain divalent group having a number of atoms of 2 to 5 composed of carbon atoms which each may be substituted, or of carbon atoms which each may be substituted and one hetero atom which may be substituted;
$-COOR^1$ and $-COOR^2$ each is the same as or different from the other and represents a carboxyl group which may be esterified or its salts are produced by reacting compounds of the formula,

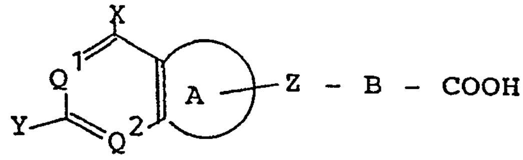

EP 0 438 261 A2

wherein A, B, $Q^1$, $Q^2$, X, Y, and Z are the same as defined above, with compounds of the formula,

$$\begin{array}{l} H_2N - CH - COOR^1 \\ \qquad\quad | \\ \qquad\quad CH_2CH_2 - COOR^2 \end{array}$$

wherein $-COOR^1$ and $-COOR^2$ are the same as defined above.

The compounds are useful as an antitumor agent.

## CONDENSED HETEROCYCLIC COMPOUDS, THEIR PRODUCTION AND USE

This invention relates to novel condensed hetero-cyclic compounds which are useful as an antitumor agent and to a production process thereof.

Folic acid and its related compounds, as a transport agent in living body of C1 units derived from formic acid or formaldehyde, play a role as a coenzyme in a great variety of enzymic reactions, such as the nucleic acid biosynthesis, amino acid-peptide metabolism and methane production systems. In the nucleic acid biosynthesis system, particularly, such compounds are essential for the C1 unit metabolism-transfer reactions in the purine synthesis and thymidine synthesis pathways. In order for folic acid to demonstrate its biological activities, normally, it is required to undergo two steps of reduction to be transformed into its active coenzyme form. As a drug substance which binds strongly to the enzyme (dihydrofolate reductase) governing the second reduction step to thereby inhibit the reduction of dihydrofolate to tetrahydrofolate, there are known asomepterine (methotrexate : MTX) and its analogous compounds. These drug substances, that act to exert damage to DNA synthesis, eventually bringing about cell death, have been developed as an antitumor agent and occupy a clinically important position. Furthermore, reports have been made of the folic acid antagonists that work through a mechanism being deifferent from the inhibition of dihydrofolate reductase, namely a tetrahydroaminopterine based antitumor agent (5,10-dideaza-5,6,7,8-tetrahydroaminopterine : DDATHF) [Journal of Medicinal Chemistry, 28, 914 (1985)] which can act mainly through a mechanism to inhibit glycineamide-ribonucleotide-transformylase being involved in the initial stage of purine biosynthetic pathway or a quinazoline-based antitumor agent (2-desamino-2-methyl-10-propargyl-5,8-dideazafolate : DMPDDF) [British Journal of Cancer, 58, 241 (1988)] which can work principally through a mechanism to inhibit thymidylate synthetase participating in the conversion of 2-deoxyuridylic acid to thymidylic acid, and others. Besides those folic acid antagonists having a basic skeleton of a condensed ring of 6-membered rings, it was also reported that the compounds having the pyrrolo[2,3-d]pyrimidine skeleton which is a condensed ring from a 6-membred ring and a 5-membered ring, exhibit antitumor activity, as well. However, there has been described that it is essential for the above-mentioned pyrrolo[2,3-d]pyrimidine compounds each to have -NH- group (a group having active proton on the N atom) at the 7-position of the compound and to bond to the side chain at the -position of the pyrrole ring. [The Japanese Patent Application No. 72235/1989].

Currently, it has been especially expected of cancer therapy to develop and create drug substances which can work through a novel mechanism of action to exhibit highly selective toxicity against cancer cells and also to achieve excellently improved therapeutic effect. MTX, an antitumor agent that can act basically through a mechanism to serve the antagnonism against folic acid, is presently put intoextensive clinical use but because of its relatively strong toxicity and inadequate efficacy against solid cancers, has failed to attain adequately satisfactory therapeutic effect.

In view of the above situations, the present inventors conducted repeatedly intensive research and as a result, found that the novel condensed heterocyclic compounds, inhibiting not less than one of the biosynthetic pathways in which folic acid and its related compounds are involved, can exhibit highly selective toxicity against various types of tumor cells and also produce excellent antitumor effect. This finding has led to completion of this invention. Namely, this ivnention is concerned with :

(1) A compound of the formula :

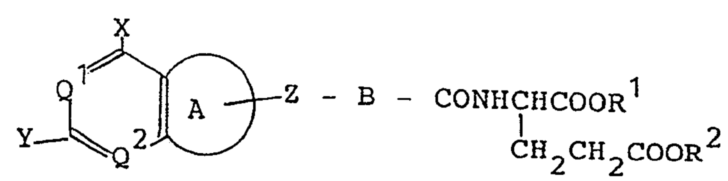

(I)

wherein the ring A is a 5-membered ring, provided that, in the case where the ring A is a pyrrole or pyrroline ring, (i) the N atom in the ring has no active proton or (ii) the ring bonds to Z at the α-position of the ring ; B is a divalent cyclic or chain group which may be substituted ; one of $Q^1$ and $Q^2$ is N, with the other being N or CH ; Y is a hydrogen or halogen atom or a group which bonds to the bonding line with a carbon, nitrogen, oxygen or sulfur atom ; X is an amino, hydroxyl or mercapto group ;

Z is a straight-chain divalent group having a number of atoms of 2 to 5 composed of carbon atoms which each may be substituted, or of carbon atoms which each may be substituted and one hetero atom which may be substituted ;

$-COOR^1$ and $-COOR^2$ each is the same as or different from the other and represents a carboxyl group which

may be esterified or its salt.

(2) A process for producing a compound described in the above item (1), which comprises reacting a compound represented by the formula :

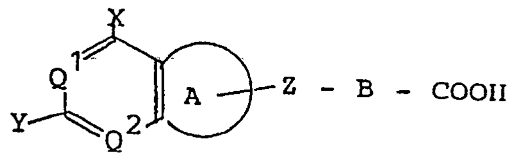

(II)

wherein the ring A is a 5-membered ring, provided that, in the case where the ring A is a pyrrole or pyrroline ring, (i) the N atom in the ring has no active proton or (ii) the ring bonds to Z at the -position of the ring ;
- B - is a divalent cyclic or chain group which may be substituted ; one of $Q^1$ and $Q^2$ is N, with the other being N or CH ; Y is a hydrogen or halogen atom or a group which bonds to the bonding line with a carbon, nitrogen, oxygen or sulfur atom ; X is an amino, hydroxyl or mercapto group ;
Z is a straight-chain divalent group having a number of atoms of 2 to 5 composed of carbon atoms which each may be substituted, or of carbon atoms which each may be substituted and one hetero atom which may be substituted ;
or its rective derivative in respect to the carboxyl group with a compound as represented by the general formula:

$$H_2N - \underset{\displaystyle |\atop\displaystyle CH_2CH_2 - COOR^2}{CH} - COOR^1 \qquad (III)$$

wherein $-COOR^1$ and $-COOR^2$ each is the same as or different from the other and represents a carboxyl group which may be esterified.

(3) An antitumor composition which contains a compound described in the above item (1) or its salt, and

(4) A compound as represented by the general formula :

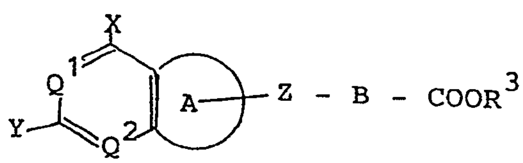

(IV)

wherein the ring A is a 5-membered ring, provided that, in the case where the ring A is a pyrrole or pyrroline ring, (i) the N atom in the ring has no active proton or (ii) the ring bonds to Z at the -position of the ring ;
B is a divalent cyclic or chain group which may be substituted ; one of $Q^1$ and $Q^2$ is N, with the other being, N or CH ; Y is a hydrogen or halogen atom or a group which bonds to the bonding line with carbon, nitrogfen, oxygen or sulfur atom ; X is an amino, hydroxyl or mercapto group ;
Y is a hydrogen atom or a hydroxyl or amino group ; Z is a straight-chain divalent group having a number of atoms of 2 to 5 composed of carbon atoms which each may be substituted, or of carbon atoms which each may substituted and one hetero atom which may be substituted ; $-COOR^3$ is a carboxyl group which may be esterified, or its salt.

In cases where in the above formulae, $Q^1$ is N and X is an amino, hydroxyl or mecapto group, the compounds (I), (II) and (IV) can exist as an equilibrium mixture with their tautomeric isomers. Illustrated below are the partial structural formulae capable of undergoing tautomerism, with the equilibria among them being shown, as well.

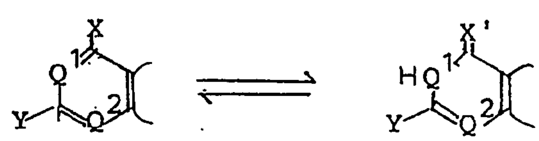

X = $NH_2$, OH, SH    X' = NH, O, S

Throughout this specification, for the purpose of convenience of expression, the amino, hydroxyl and mercapto forms are to be described, with the corresponding designations being adopted, and in either case, their tautomers or the imino, oxo and thiooxo forms are understood to be included in the scope of this invention.

In the compounds (I) of this invention, furthermore, the presence of a plural number of asymmetric centers is possible, but except that the absolute configuration of the asymmetric carbon atom in the side chain derived from the glutamic acid moiety is S(L), the absolute configurations of other asymmetric centers may be either of S, R and a mixture of RS. In such a case, a plurality of diastereomers exist, and they can be easily separated by conventional separation and purification means, if necessary.

All the above-described diastereomers that can be separated by such procedures are included in the scope of this invention.

Referring to the above formulae, the ring A is a 5-membered ring and includes for example 5-membered rings being composed of carbon atoms alone or both carbon atoms and one hetero atom (nitrogen, oxygen or sulfur atom), which groups may be substituted, however, in the case where the ring is pyrrole or pyrroline ring, (i) the N atom in the ring has no active proton or (ii) the ring bonds to Z at α-position of the ring. As examples of the said ring, there may be mentioned cyclopentadiene, cyclopentene, furan, dihydrofuran, thiophene, dihydrothiophene, thiophene-1-oxide, dihydrothiophene-1-oxide, thiophene-1,1-dioxide, dihydrothiophene-1,1-dioxide, N-substituted pyrrole and N-substituted pyrroline.

These rings may have one to two substituents at their replacable positions, and examples of such substituents include $C_{1-3}$ alkyl groups (e.g., methyl, ethyl, propyl and isopropyl groups), $C_{2-3}$ alkenyl groups (e.g., vinyl, 1-methylvinyl, 1-propenyl, allyl and allenyl groups), $C_{2-3}$ alkynyl groups (e.g., ethynyl, 1-propynyl and propargyl groups), $C_{3-6}$ cycloalkyl groups (e.g., cyclopropyl group), halogen atoms (e.g., fluorine, chlorine, bromine and iodine), $C_{1-4}$ alkanoyl groups (e.g., formyl, acetyl, propionyl, butyryl and isobutyryl groups), benzoyl group, substituted benzoyl groups (e.g., p-chlorobenzoul, p-methoxybenzoyl and 3,4,5-triemthoxybenzoyl groups), cyano group, carboxyl group, carbamoyul group, nitro group, hydroxyl group, hydroxy-$C_{1-4}$ alkyl groups (e.g., hydroxymethyl group and hydroxyethyl group), $C_{1-4}$ alkoxy-$C_{1-4}$ alkyl groups (e.g., methoxymethyl group, ethoyxmethyl group, methoxyethyl group and ethoxyethyl group), $C_{1-3}$ alkoxy groups (e.g., methoxy, ethoxy and propoxy groups), mercapto group, $C_{1-3}$ alkylthio groups (e.g., methylthio, ethylthio and propylthio groups), amino group, $C_{1-4}$ substituted amino groups (e.g., methylamino, ethylamino, dimethylamino and diehtylamino groups), $C_{1-4}$ alkanoylamino groups (e.g., formamide and acetamide groups), and the like.

In the case where the ring A is pyrrole or pyrroline ring and there is no active proton on the N atom, the N-substitued moieties in the N-substituted pyrrole and N-substituted pyrroline rings include, for example, those groups mentioned in the above, such as $C_{1-3}$ alkyl groups, $C_{2-3}$ alkenyl groups, cyclopropyl group, $C_{1-4}$alkanoyl groups, benzoyl group, substituted benzoyl groups, hydroxyethyl group, methoxyethyl group and ethoxyethyl group, as well as phenyl group, substituted phenyl groups (e.g., p-chlorophenyl, p-ethoxyphenyl and 3,4,5-trimethoxyphenyl groups) benzyl group or substituted benzyl groups (e.g., p-chlorobenzyl, p-methoxybenzyl and diphenylmethyl groups). The bonding between the ring A and -Z- may take place at any feasible positions, however, in the case where the ring A is pyrrole or pyrroline ring and there is no active proton on the N atom, the bonding take place at -position of the ring A. In the cases that the ring A is N-substituted pyrrole or N-substituted pyrroline ring, the bonding may occur at the N-substituted moiety.

The divalent cyclic group in the divalent cyclic group which may have a substituent, as represented by B, is desirably a divalent 5- or 6-membered cyclic group which may contain one to three of heteroatoms (e.g., N, O and S) in the ring, with their two linkages preferably extending from the positions not mutually adjacent in the rings.

As examples of the said divalent 5- membered cyclic hydrocarbon or hetero ring group represented by B, there are mentioned 1,3- or 3,5-cyclopentadiene-1,3-ylene, cyclopentene-(1,3, 1,4- or 3,5-)ylene, cyclopentane-1,3-ylene, thiophene-(2,4-, 2,5- or 3,4-)ylene, furan-(2,4-, 2,5- or 3,4-)ylene, pyrrole-(1,3-, 2,4-, 2,5-or 3,4-)ylene, thiazole-(2,4- or 2,5-)ylene, imidazole-(1,4-, 2,4- or 2,5-)ylene, thiadiazole-2,5-ylene, or their partially reduced forms or completely reduced forms. Examples of the divalent 6-membered cyclic hydrocarbon or hetero groups represented by B include phenyl-(1,3- or 1,4-)ylene, cyclohexane-(1,3- or 1,4-)ylene, cyclohexene-(1,3-, 1,4, 1,5-, 3,5- or 3,6-)ylene, 1,3-cyclohexadiene-(1,3-, 1,4, 1,5-, 2,4-, 2,5- or 2,6-)ylene, 1,4-cyclohexadiene(1,3-, 1,4- or 1,5-)ylene, pyridine-(2,4-, 2,5-, 2,6- or 3,5-)ylene, pyran-(2,4-, 2,5-, 2,6-, 3,5-, 3,6- or 4,6-)- ylene, pyrazine-(2,5-or 2,6-)ylene, pyrimidine-(2,4- or 2,5-)ylene, pyridazine-3,5ylene, or their partially reduced forms or completely reduced forms. As divalent cyclic group represented by B, phenyl-1,4-ylene and thiophene-2,5-ylene are particularly preferable.

The divalent chain group which may have a substituent, as represented by B, preferably are divalent $C_{2-4}$ chain hydrocarbon groups, and include, for example, a $C_{2-4}$ alkylene, $C_{2-4}$ alkenylene or $C_{2-4}$ alkynylene group, such as ethylene, ethenylene, ethynylene, trimethylene, propenylene, propynylene, propadienylne, tetramethylene, butenylene, butynylene, butanedienylene, etc.

The divalent cyclic or lower chain hydrocarbon residue as represented by B may have one or two substituents at its replacable positions. Examples of the said substituents include $C_{1-3}$ alkyl groups (e.g., methyl, ethyl, propyl, and isopropyl groups), $C_{2-3}$ alkenyl groups (e.g., vinyl, 1-methylvinyl, 1-propenyl, allyl and allenyl groups), $C_{2-3}$ alkynyl groups (e.g., ethynyl, 1-propenyl and propargyl groups), halogens (e.g, chlorine, bromine, fluorine and iodine), hydroxyl, methoxy, dimethyl- amino, trifluoromethyl, oxo, formyl, methoxymethyl and 2-ethoxyethyl.

The group represented by Y, which bonds to the bonding line in the formula with carbon, nitrogen or sulfur atom may be cyano group, carboxyl group, carbamoyl group, amino group, nitro group, hydroxyl group, mercapto group or a lower hydrocarbon group, such as a $C_{1-3}$ alkyl group (e.g., methyl, ethyl, propyl and isopropyl goups), a $C_{2-3}$ alkenyl group (e.g., vinyl, 1-methylvinyl, 1-propenyl, allyl or allenyl groups), a $C_{2-3}$ alkynyl group (e.g., ethynyl, 1- propynyl or propargyl group) and cyclopropyl group ;
an $C_{6-10}$ aryl group, such as phenyl or naphthyl group ; and 5- or 6-membered heterocyclic groups, such as pyrrolyl, imidazolyl, pyrazolyl, thienyl, furyl, thiazolyl, thiadiazolyl, oxazolyl, oxadiazolyl, pyridyl, pyranyl, pyrazinyl, pyrimidinyl, pyridazinyl or their partially reduced forms or completely reduced forms, dioxoranyl, piperizino, morpholino, N-methyl- piperazinyl, N-ethylpiperazinyl and dioxanyl. In cases where Y is a lower hydrocarbon group, an aryl or 5- or 6-membered heterocyclic group, such group may have one to two substituents, and examples of such substituents include a $C_{1-3}$ alkyl group (e.g., methyl, ethyl, propyl or isopropyl group), a $C_{2-3}$ alkenyl group (e.g., vinyl, 1-methylvinyl, 1-propenyl, allyl, or allenyl group), a $C_{2-3}$ alkynyl group (e.g., ethynyl, 1-propynyl or propargyl group) or cyclopropyl group, as well as, fluorine, hydroxyl, oxo, methoxy, dimethylamino, diethylamino, trifluoromethyl, formyl, hydroxyemthyl, 2-hydroxyethyl, methoxymethyl 2-ethoxyethyl.

The halgoen atom represented by Y includes, for example, fluorine, chlorine, bromine and iodine. The group represented by Y, which bonds to the bonding line with carbon, nitrogen or sulfur atom, may be an alkoxy group, alkylthio group, alkanoylamino group or alkanoyloxy group, and the alkyl moiety in such groups includes, as such, those as exemplified in the case of Y being a lower hydrocarbon group. The group represented by Y, which bonds to the bonding line with carbon, nitrogen or sulfur atom may be an aryloxy group, arylthio group, aroylamino group and aroyloxy group, and the aryl moiety in such groups includes, for example, phenyl or naphthyl group, etc. Furthermore, the group represented by Y, which bonds to the bonding line with carbon, nitrogen or sulfur atom, may be a heterocyclic-oxy, heterocyclic-thio, heterocyclic-carbonylamino or heterocyclic-carbonyloxy group wherein the heterocyclic moiety in such groups includes, as such, those as exemplified in the case of Y representing a 5- or 6-membered heterocyclic group. The group represented by Y, which bonds to the bonding line, may be a substituted amino group, such as mono-substituted or di-substituted amino group, wherein such substituted moieties include, as such, those as exemplified above in the case of Y being a lower hydrocarbon group, as well as an aryl, or 5- or 6-membered heterocyclic group.

The carboxyl group which may be esterified, as represented by $-COOR^1$, $-COOR^2$ and $-COOR^3$, include for example carboxyl group being esterified with a $C_{1-5}$ lower alkyl group, a benzyl group which may be substituted or phenyl groups which may be substituted. As the said lower alkyl groups, there may be mentioned, for example, methyl, ethyl, propyl, isopropyl, n-butyl, iso-butyl, sec-butyl, tert-butyl, n-pentyl, isopentyl, sec-pentyl, neo-pentyl and tert-pentyl, and the said benzyl which may be substituted includes, for example, benzyl, nitrobenzyl and methoxybenzyl, while the said phenyl which may be substituted includes, for example, phenyl, nitrophenyl and methoxyphenyl.

In the divalent chain group having a number of atoms of 2 to 5 composed of carbon atoms which may be substituted, or of carbon atoms and one heteroatom (nitrogen, oxygen or sulfur atom) which may be substituted, as represented by Z, the group composed of carbon atoms includes, for example, $C_{2-5}$ alkylene groups such as ethylene, trimethylene, tetramethylene and pentamethylene ; $C_{2-5}$ alkenylene groups, such as vinylene, propenylene, 1- or 2-butenylene, butadienylene, 1- or 2-pentenylene, and 1,3-or 1,4-penta- dienylene ; and $C_{2-5}$ alkynylene groups such as ethynylene, 1- or 2-propynylene, 1- or 2-butylenylene, and 1-, 2- or 3-pentynylene, while the group composed of carbon atoms and one heteroatom (nitrogen, oxygen or sulfur atom) includes, for example, a group represented by the formula $-Z^1-Z^2-Z^3-$ [wherein $Z^1$ and $Z^3$ each independently represents a divalent $C_{1-4}$ lower hydrocarbon group (provided that the total number of carbon atoms in $Z^1$ and $Z^3$ is one to four) which may have a bonding linkage or may be substituted ; $Z^2$ is -O- or a group of the formula $-S(O)_n-$ (wherein n is an integer of 0 to 2) or the formula $R^4$ -N- (wherein $R^4$ is a hydrogen atom or a lower hydrocabron residue which may have a substituent)]. As the divalent lower hydrocarbon group which may be substituted, as represented by $Z^1$ and $Z^3$, there may be used, for example, a $C_{1-4}$ alkylene group such as methylene, ethylene, trimethylene and tetramethylene, a $C_{2-4}$ alkenylene group such as vinylene, propenylene, 1- or 2-butenylene or butadienylene, or a $C_{2-4}$ alkynylene group such as ethynylene, 1- or 2-propynylene or 1- or 2-butynylene, while the lower hydrocarbon group which may be substituted, as represented by $R^4$, includes for example a $C_{1-3}$ alkyl group (e.g., methyl, ethyl, propyl or isopropyl groups), a $C_{2-3}$ alkenyl group (e.g., vinyl, 1-methylvinyl, 1-propenyl, allyl or allenyl groups), a $C_{2-3}$ alkynyl group (e.g., ethynyl, 1-propynyl or propargyl

groups) or cyclopropyl group. The carbon atoms or a heteroatom which compose of divalent group represented by Z, or a lower hydrocarbon group represented by $Z^1$ , $Z^3$ or $R^4$ may have one to two of substituent which includes, for example, a $C_{1-3}$ alkyl group (e.g., methyl, ethyl, propyl or isopropyl group), a $C_{2-3}$ alkenyl group (e.g., vinyl, 1-methylvinyl, 1-propenyl, allyl or allenyl group), a $C_{2-3}$ alkynyl group (e.g., ethynyl, 1-propynyl or propargyl group) or cyclopropyl group, as well as fluorine, hydroxyl, oxo, methoxy, dimethylamino, diethylamino, trifluoromethyl, formyl, hydroxymethyl, 2hydroxyethyl, methoxymethyl and 2-ethoxyethyl.

Below described is the process for producing the compounds (I) of this invention or their salts.

The compounds (I) or their salts can be obtained by acylating a glutamic acid deerivative as represented by the formula (III) with a carboxylic acid represented by the formula (II) or its reactive derivative in regard to the carboxyl group. The above-mentioned acylating means includes, for example, a procedure of acylating the compound (III) with the compound (II) or its reactive derivative in the presence of carbodiimides, diphenylphosphoric azide or diethyl cyanophosphate. The used amount of the compound (III) usually is in the range of about 1 to 20 mole equivalents against the compound (II) or its reactive derivative, preferably about 1 to 5 mole equivalents. The above carbodiimides etc. may be used ordinarily at a ratio of about 1 to 25 mole equivalents, preferably about 1 to 5 mole equivalents.

As the said carbodiimides, dicyclocarbodiimide is desirable from the standpoint of practical use, and in addition to this, there may be utilized other carbodiimides, such as diphenylcarbodiimide, di-o-tolylcarbodiimide, di-p-tolylcarbodiimide, di-tert-butylcarbodiimide, 1-cyclohexyl-3-(2-morpholinoethyl)carbodiimide, 1-cyclohexyl-3-(4-diethylaminocyclohexyl)carbodiimide, 1-ethyl-3-(2-diethylaminopropyl)carbodiimide and 1-ethyl-3-(3-diethylaminopropyl)carbodiimide. The said acylation reaction is preferably carried out in the presence of an appropriate solvent, and as the solvent, use is made for example of water, alcohols (e.g., methanol and ethanol), ethers (e.g., dimethyl ether, diethyl ether, tetrahydrofuran, dioxane, monoglyme and diglyme), nitriles (e.g., acetonitrile), esters (e.g., ethyl acetate), halogenated hydrocarbons (e.g., dichloromethane, chloroform and carbon tetrachloride), aromatic hdyrocarbons (e.g., benzene, toluene and xylene), acetone, nitromethane, pyridine, dimethylsulfoxide, dimethylformamide, hexamethylphosphoramide, sulfolane or suitable solvent mixtures thereof. This reaction can be conducted by allowing the reaction to proceed in a solvent at a pH in the range of about 2 to 14, preferably about 6 to 9 and at a reaction temperature in the region of about -10°C to the boiling point (up to about 100°C) of the used reaction solvent, preferably about 0 to 50°C, for about 1 to 100 hours. The pH value of the reaction solution is suitably adjusted, for example, with acids (e.g., hydrochloric acid, sulfuric acid, phosphoric acid, nitric acid and acetic acid), bases (e.g., sodium methylate, sodium ethylate, sodium hydroxide, potassium hydroxide, barium hydroxide, lithium hydroxide, sodium carbonate, potassium carbonate, barium carbonate, calcium carbonate, sodium hydrogencarbonate, triemthylamine, triethylamine, triethanolamine and pyridine) or buffers (e.g., phosphate buffer, borate buffer and acetate buffer), if necessary. This reaction can be allowed to proceed more advantageously by utilizing catalysts capable of accelerating the reaction. Such catalysts include, for example, base catalysts and acid catalysts. As the said base catalysts, there may be mentioned, for example, tertiary amines (e.g., aliphatic tertiary amines, such as triethylamine ; aromatic tertiary amines, such as pyridine, alpha-, beta- or gamma-picoline, 2,6-lutidine, 4-dimethylaminopyridine, 4-(1-pyrrolidinyl)-pyridine, dimethylaniline and diethylaniline), while the acid catalysts include, for example, Lewis acids (e.g., anhydrous zinc chloride, anhydrous aluminum chloride ($AlCl_3$), anhydrous ferric chloride, titanium tetrachloride ($TiCl_4$), tin tetrachloride ($SnCl_4$), antimony pentachloride, cobalt chloride, cupric chloride, and boron trifluoride etherate). Among the above-cited catalysts, in many cases, 4-dimethyl- aminopyridine or 4-(1-pyrrolidinyl)pyridine is preferred. The used amount of the catalysts desirably is in the neighborhood of the catalytic quantities which can permit the acylation to be accelerated, and normally ranges from about 0.01 to 10 mole equivalents against the compound (II), preferably from about 0.1 to 1 mole equivalent. The reactive derivatives of the carboxylic acids (II) in regard to the carboxylic group include, for example, derviatives of the compounds (II), such as their acid halides (e.g., fluorides, chlorides, bromides and iodides), their acid anhydrides (e.g., iodo-acetic anhydrides and isobutyric anhydrides), their mixed acid anhydrides with lower mono-alkyl carbonates (e.g., monomethyl carbonate, monoethyl carbonate, monopropyl carbonate, monoisopropyl carbonate, monobutyl carbonate, monoisobutyl carbonate, mono-sec-butyl carbonate and mono-tert-butyl carbonate), their mixed-acid anhydrides with active esters (e.g., cyanomethyl esters, ethoxycarbonylmethyl esters, methoxymethyl esters, phenyl esters, o-nitrophenyl esters, p-nitrophenyl esters, p-carbomethoxyphenyl esters, p-cyanophenyl esters and phenylthio esters), their acid azides, their mixed-acid anhydrides with phosphoric acid esters (e.g., dimethyl phosphate, diethyl phosphate, dibenzyl phosphate and diphenyl phosphate) and their mixed-acid anhydrides with phosphorous acid esters (e.g., dimethyl phosphite, diethyl phosphite, dibenzyl phosphite and diphenyl phosphite). In the acylation means with use of such reactive derivatives, the reaction conditions such as the solvent, catlayst and reaction temperature, are the same as described previously in the case of acylation being carried out in the presence of carbodiimides.

In producing the compound (I-1), or its salt, where -$COOR^1$ and -$COOR^2$ are a carboxyl group, out of the

compound (I) or its salt, it is desirable to react the compound (III) where -$COOR^1$ and -$COOR^2$ are an esterified carboxyl group with the compound (II) or its reactive derivative in regardto the carboxyl group, followed by the per se known decomposition or catalytic reduction reaction to conduct deesterification. The said decomposition reaction includes, for example, a hydrolysis reaction under basic conditions (Method A), a hydrolysis reaction under acid conditions (Method B-1) and a decomposition reaction under acid, nonaqueous conditions (Method B-2). Examples of the base which can be used in the Method A include metal alkoxides, such as sodium methoxide, sodium ethoxide, sodium butoxide and potassium butoxide, metal hydroxides, such as sodium hydroxide, potassium hydroxide, lithium hydroxide and barium hydroxide, and amines, such as ammonia, triethylamine and pyridine, and as the acid being usable in the Method B-1, there may be mentioned mineral acids, such as hydrochloric acid, hydrobromic acid, sulfuric acid, nitric acid and phosphoric acid, and organic acids, such as trifluoroacetic acid, trichloroacetic acid, methanesulfonic acid, benzene- sulfonic acid, p-toluenesulfonic acid and camphorsulfonic acid, while the catalyst being utilizable in the Method B-2 incldues, for examle, mineral acids, such as hydrochloric acid, hydrobromic acid, perchloric acid, sulfuric acid, nitric acid and phosphoric acid, organic acids, such as trifluoroacetic acid, trichloroacetic acid, methansuflonic acid, benzenesulfonic acid, p-toluenesulfonic acid and camphorsulfonmic acid, and Lewis acids, such as anhydrous zinc chloride, anhydrous aluminum chloride ($AlCl_3$), anhydrous ferric chloride, titanium tetrachloride ($TiCl_4$), tin tetrachloride ($SnCl_4$), antimony pentachloride, cobalt chloride, cupric chloride and boron trifluoride etherate. Any of the decomposition reactions is carried out in an appropriate solvent at a temperature in the range of 0°C to the boiling point of the used solvent, preferably 10 to 80°C for 30 min to 2 days. Referring to the reaction solvent, usable in the case of the Methods A and B-1 are for example water, methanol, ethanol, proapnol,butanol, ethylene glycol, methoxyethanol, ethoxyethanol, tetrahydrofuran, dioxane, monoglyme, diglyme, pyridine, dimethylformamide, dimethylsulfoxide, sulfolane or suitable solvent mixtures thereof, whereaas in the case of the Method B-2, there are utilized for example ethyl acetate, dimethyl ether, diethyl ether, tetrahydrofuran, dioxane, monoglyme, diglyme, dichloromethane, chloroform, carbon tetrachloride, aceto- nitrile, benzene, toluene, xylene, nitromethane, pyridine or suitabnle solvent mixtures thereof. The said catalytic reduction reaction (Method C) is carried out in a suitable solvent at a temperature in the range of about -40°C to the boiling point of the used solvent, more preferably about 0° to 50°C. The usable solvent includes, for example, water, alcohols (e.g., methanol, ethanol, propanol, isopropanol, butyl alcohol, sec-butyl alcohol, tert-butyl alcohol, ethylene glycol, methoxyethanol and ethoxyethanol), acetates (e.g., methyl acetate and ethyl acetate), ethers (e.g., dimethyl ether, diethyl ether, tetrahydrofuran, dioxane, monoglyme and diglyme), aromatic hydrocarbons (e.g., benzene, toluene and xylene), pyridine, dimethylformamide and suitable solvent mixtures thereof. As the catalyst for the catalytic reduciton, there may be used, for example, palladium, platinum, rhodium and Raney nickel. On the occasion of utilizing such catalysts, addition of minute quantities of acetic acid, trifluoroacetic acid, hydrochloric acid, sulfuric acid, etc. can in some instances permit the reaction to proceed advantageously. It depends upon the properties of -$COOR^1$ and -$COOR^2$ which reaction should be employed for the conversion to the compound (I1) ; normally, the Method A or B-1 is applicable advantageously, when -$COOR^1$ and -$COOR^2$ are carboxyl groups esterified with methyl, ethyl, propyl, butyl, sec-butyl, phenyl or substituted phenyls, and the Method B-2 can be applied favorably in the case of -$COOR^1$ and -$COOR^2$ being carboxyl groups esterified with isopropyl or tert-butyl group, whereas the Method B-1 or C is utilizable advantageously in the case of -$COOR^1$ and -$COOR^2$ being carboxyl groups esterified with benzyl or substitued benzyl groups. In cases where -$COOR^3$ and -$COOR^4$ each is different from the other, the above-mentioned Methods A, B-1, B-2 and C may be suitably combined.

Described in the following is the procedure of producing the starting compound (II) :

The compound (II) can be produced for example by means of the following reaction steps :

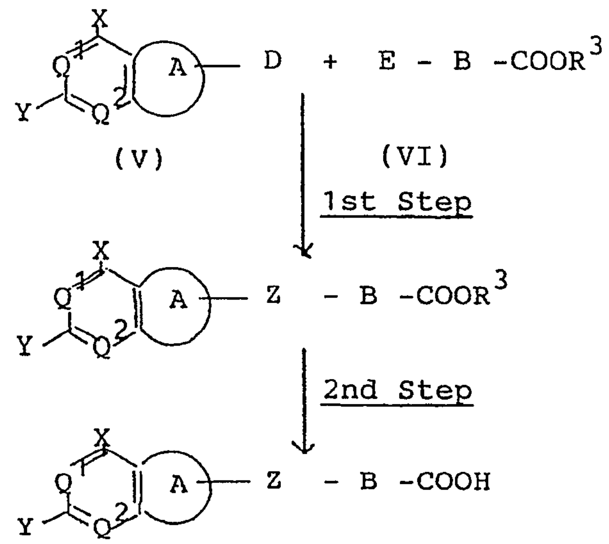


(IV)

(II)

In the above steps, the ring A, B, $-COOR^3$, $Q^1$, $Q^2$, X, Y and Z are as defined hereinbefore ; D and E are groups being capable of combining with each other to form Z.

In the above reaction steps, the covalent bond can allowed to be formed between D and E to thereby produce a straight-chain divalent group having a number of atoms of 2 to 5 composed of carbon atoms which each may have a substituent or carbon atoms and one heteroatom.

Referring to the synthetic method which permits formation of the covalent bond between the compound (V) and compound (VI), the compound (V) and the compound (VI) can be subjected to the so-called reaction causing the carbon-carbon bond, followed by a reduction reaction, if necessary, to thereby produce the compound (IV), in the case of the group being composed of carbon atoms which each may have a substituent, for example, in cases where D is D' =

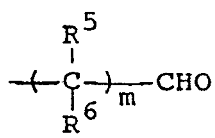 and E is E' = H 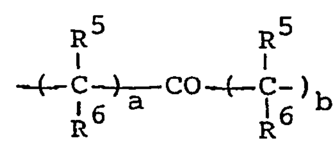, $G_3P$ =

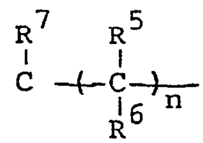 or 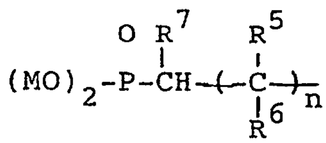, and <u>vice</u> <u>versa</u> D=E'

and E=D'.

In the above formulae, <u>a</u>, <u>b</u>, <u>m</u>, <u>n</u> (= <u>a</u>+<u>b</u>) and <u>m</u>+<u>n</u> each is an integer in the range of 0 to 3 ; G is phenyl, butyl or cyclohexyl ; M is ethyl or phenyl ; $R^5$, $R^6$ and $R^7$, each being the same as or different from the other, represent a bonding linkage, a hydrogen atom or a substituent in the lower hydrocarbon groups described in detail for the groups $Z^1$, $Z^2$ and $R^4$, and may be different from the other in repeating units <u>m</u> and <u>n</u>.

In the case of Z being a group composed of Z = $-Z^1-Z^2-Z^3-$,

for example, in cases where D is $D^1$ = 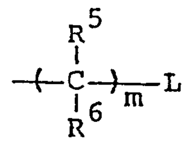 and

E is $E^1$ = 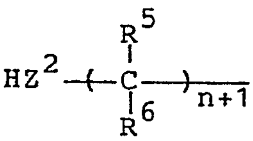, and vice versa $D=E^1$ and $E=D^1$,

the so-called alkylation reaction is employed; when

D is $D^2$ = 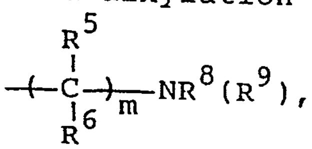, with E being $E^2$ = 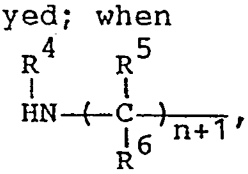,

and vice versa $D=E^2$ and $E=D^2$, for example, the so-called amine exchange reaction (glamine decomposition reaction) is advanatageously used ; and in cases where D is $D^3$=

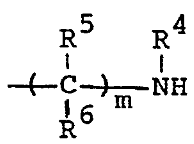 and E is $E^3$ = 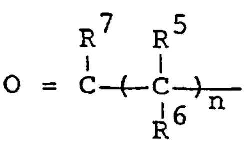, and vice versa

$D=E^3$ and $E=D^3$, for example, use is made of a procedure which comprises allowing a Schiff base to be formed, followed by reduction, if neessary, or subjecting directly to a reductive alkylation reaction.

In the above formulae, m, n, m+n, $R^4$, $R^5$, $R^6$, $R^7$ and $Z^2$ are as defined hereinbefore ; L is a removable group ; and $R^8$ and $R^9$ each is the same as or different from the other and represents a hydrogen atom or a hydrocarbon group which may be substituted. The removable group as represented by L includes, for example, halogen atoms (e.g., chlorine, bromine and iodine atoms) or removabale groups derived easily from the hydroxyl group (e.g., methanesulfonyloxy, benzenesulfonyloxy, p-toluenesulfonyloxy and trifluoromethanesulfonyl groups). The hydrocarbon group represented by $R^8$ and $R^9$ may have substituents, and $R^8$ and $R^9$ both may cooperate with the adjacent nitrogen atom to form a cyclic amino group. The hydrocarbon groups represented by $R^8$ and $R^9$ inlcude, for example, alkyl groups of $C_1$ to $C_{18}$ (e.g., methyl, ethyl, propyl, isopropyl, butyl, isobutyl, sec-butyl, tert-butyl, pentyl, isopentyl, hexyl, isohexyl, heptyl, octyl, nonyl, decyl, undecyl, dodecyl, tradecyl, hexadecyl, octadecyl, 1,2-dimethylpropyl, 1-ethylpropyl, 1,2,2-tri- methylpropyl, 1-propylbutyl and 2-ethylhexyl groups), alkenyl groups of $C_1$ to $C_{12}$ (e.g., vinyl, allyl, 1-methylvinyl, 2-methylvinyl, 1-octenyl and 1-decenyl groups), cycloalkyl groups of $C_3$ to $C_{12}$ (e.g., cyclopropyl, cyclobutyl, cyclopentyl, cyclohexyl, cycloheptyl, cyclooctyl and admantyl groups), cycloalkenyl groups of $C_3$ to $C_8$ (e.g., cyclo- pentenyl, cyclohexenyl, cyclo- heptenyl, cyclooctenyl, cyclopentadienyl, cyclohexadienyl, cycloheptadienyl and cyclooctadienyl groups), aralkyl groups of $C_7$ to $C_{13}$ (e.g., benzyl, alpha-methylbenzyl, phenethyl and diphenylmethyl groups), and aryl groups of $C_6$ to $C_{10}$ (e.g., phenyl, alpha-naphthyl and beta-naphthyl groups). Preferred examples of the cyclic amino group which $R^8$ and $R^9$ cooperate with the adjacent nitrogen atom to form include 4- to 10-membered rings, such as azetidinyl, pyrrolidinyl, pyrrolinyl, pyrrolyl, imidazolyl, pyrazolyl, imidazolinyl, piperidino, morpholino, dihydropyridyl, tetrahydropyridyl, N-methylpiperadinyl, N-ethylpiperazinyl, azacycloheptyl, azacyclooctyl, isoindolyl, indolyl, indolinyl, 2-iso- indolinyl, azacyclononyl or azacyclodecyl group, etc.

Such hydrocarbon groups as represented by $R^8$ and $R^9$ as well as such cyclic amino groups as formed by $R^8$ and $R^9$ in cooperation with the adjacent nitrogen atom may have one to two substituents. As the said substituent, there may be mentioned, for example, alkyl groups of $C_1$ to $C_4$ (e.g., methyl, ethyl, propyl, isopropyl, butyl, isobutyl, sec-butyl and tert-butyl groups), alkoxy groups of about $C_1$ to $C_4$ (e.g., methoxy, ethopxy, propoxy, isopropoxy, n-butoxy, iso-butoxy, sec-butoxy and tert-butoxy groups), alkanoyl groups of about $C_1$ to $C_4$ (e.g., formyl, acetyl, propionyl, n-butyryl and iso-butyryl groups), alkanoyloxy groups of about $C_1$ to $C_4$ (e.g., formyloxy, acetyloxy, propionyloxy, n-butyryloxy and iso-butyryloxy groups), carboxyl group, alkoxycarbonyl groups of about $C_2$ to $C_4$ (e.g., methoxy- carbonyl, ethoxycarbonyl, n-propoxycarbonyl, isopropoxy- carbonyl, n-butoxycarbonyl, isobutoxycarbonyl and tert- butoxycarbonyl groups), halogen atoms (e.g., fluorine, chlorine, bromine and iodine), hydroxyl group, nitro group, cyano group, triluforoemthyl group, amino group, mono- substituted amino groups (e.g., mmethyl- amino, ethylamino, propylamino, isopropylamino and butylamino groups),

disubstituted amino groups (e.g., dimethylamino, diethyl- amino, dipropylamino, diisopropylamino and dibutylamino groups), alkanoylamido groups (e.g., formamido, acetamido, trifluoroacetamido, propionylamido, butrylamido and isobutyrylamido groups), carbamoyl group, N-substituted carbamoyl groups (e.g., N-methylcarbamoyl, N-ethylcarbamoyl, N-propylcarbamoyl, N-isopropylcarbamoyl and N-butylcarbamoyl groups), N,N-disubstituted carbamoyl groups (e.g., N,N-dimethylcarbamoyl, N,N,-diethylcarbamoyl, N,N-dipropylcarbamoyl, N,N-dibutylcarbamoyl, 1-atiridinylcarbonyl, 1azetidinylcarbonyl, 1-pyrrolidinylcarbonyl, 1-piperidinylcarnbonyl, N-methylpiperadinylcarbonyl and morpholinocarbonyl groups), carbamoylamino group, N-substituted carbamoylamino groups (e.g., N-methylcarbamoylamino, N-ethylcarbamoylamino, N-propylcarbamoylamino, N-isopropylcarbamoylamino and N-butylcarbamoylamino groups), N,N-disubstituted carbamoylamino groups (e.g., N,N-disubstituted carbamoylamino groups (e.g., N,N-diemthylcarbamoylamino, N,N-diethylaminocarbamoyl, N,N-dipropylaminocarbamoyl, N,Ndibutylaminocarbamoyl, 1-atilidinylcarbamoylamino, 1-azetidinylcarbonylamino, 1-pyrrolidinylcarbonylamino, 1-piperidinylcarbonylamino, N-methylpiperadinylcarbonylamino, and morpholinocarbonylamino groups), mercapto group, sulfo group, sulfino group, sulfono group, sulfamoyl group, N-substituted sulfamoyl groups (e.g., N-methylsulfamoyl, Nethylsulfamoyl, N-propylsulfamoyl, N-isopropylsulfamoyl and N-butylsulfamoyl groups), N,N-disubstituted sulfamoyl groups (e.g., dimethylsulfamoyl, N,N-diethylsulfamoyl, N,Ndipropylsulfamoyl, N,N-dibutylsulfmoyl, 1-pyrrolidinylsulfonyl, 1-piperadinylsulfonyl adn morpholinosulfonyl groups), alkylthio groups of about $C_1$ to $C_4$ (e.g., methylthio, ethylthio, propylthio, isopropylthio, n-butylthio, sec-butylthio and tert-butylthio groups), alkylsulfinyl groups of about $C_1$ to $C_4$ (e.g., methylsulfinyl, ethyl- sulfinyl, propylsulfinyl and butylsulfinyl groups) and alkylsulfonyl groups of about $C_1$ to $C_4$ (e.g., methyl- sulfonyl, ethylsulfonyl, propylsulfonyl and butylsulfonyl groups).

Below given is the detailed description of the 1st reaction step :

For the condensation reaction through the formation of the carbon-carbon bonding, the known reactions (e.g., aldol reaction, Reformatsky reaction and Wittig reaction) are employable, while as the reduction reaction, normally, there is advantageously used the previously mentioned catalytic reduction reaction (Method C). When the aldol reaction is employed as a condensation reaction, the base catalyst which is usable includes, for example, metal hydroxides, such as sodium hydroxide, potassium hydroxide, lithium hydroxide and barium hydroxide, metal alkoxides, such as sodium methoxide, sodium ethoxide and potassium tert-butoxide, metal amides, such as sodium amide and lithium diisopropylamide, metal hydrides, such as sodium hydride and potassium hydride, organic metal compounds, such as phenyllithium and butyrllithium, and amines, such as triethylamine, pyridine, α-, β - or γ-picoline, 2,6-lutidine, 4-dimethylaminopyridine, 4-(1-pyrrolidinyl)pyridine, diemthylaniline and diethylaniline, while as the acid catalyst, there are mentioned for example mineral acids, such as hydrochloric acid, sulfuric acid, nitric acid, phosphoric acid and boric acid, and organic acids, such as oxalic acid, tartaric acid, acetic acid, trifluoroacetic acid, methanesulfonic acid, benzenesulfonic acid, p-toluenesulfonic acid and camphorsulfonic acid. According to the known procedure (Ei-Ichi Negishi, "Organometallics in Organic Synthesis", vol. 1, John Wiley & Sons, New York, Chichester, Brisbane, Tronto), the ketone form is derived into the silyl enol ether form, which is then subjected to a condensation reaction with an aldehyde or its equivalent in the presence of Lewis acid (e.g., anhydrous zinc chloride, anhydrous aluminum chloride ($AlCl_3$), anhydrous ferric chloride, titanium chloride ($TiCl_4$), stannic chloride ($SnCl_4$), antimony pentachloride, cobalt chloride, cupric chloride and boron trifluoride etherate), or the ketone form can be treated with metal trifurate (e.g., dialkylboron trifurate and tin (II) trifurate) and amines (e.g., triethyl- amine, pyridine, α-, β- or γ-picoline, 2,6--lutidine, 4-dimethylaminopyridine, 4-(1-pyrrolidinyl)pyridine, dimethylaniline and diethylaniline) to convert into the enolate, followed by a condensation reaction with an aldehyde or its equivalent. The condensation reaction can be carried out in an appropriate solvent at a temperature in the range of -100°C to the boiling point of the used solvent, preferably -78 to 100°C, for 1 min to 3 days. As the solvent, there may be used, for example, water, liquid ammonia, alcohols (e.g., methanol, ethanol, propanol, iso-propanol, butyl alcohol, sec-butyl alcohol, tert-butyl alcohol, ethylene glycol, methoxyethanol and ethoxyethanol), ethers (e.g., dimethyl ether, diethyl ether, tetrahydrofuran, dioxane, monoglyme, diglyme), halogenated hydrocarbons (e.g. dichloromethane, chloroform, carbon tetrachloride,), aliphatic hydrocarbons (e.g., pentane, hexane, heptane), aromatic hydrocarbons (e.g., benzene, toluene, xylene), dimethylformamide, dimethylsufoxide, hexamethylphosphramide, sulfolane and suitable solvent mixtures thereof. In the case of employing Wittig reaction as a condensation reaction, usable reagents are, for example, metal hydroxides such as sodium hydroxide, potassium hydroxide, lithium hydroxide, barium hydroxide etc., metal alkoxides such as sodium methoxide, sodium ethoxide, potassium tert-butoxide, sodium amide, lithium doisopropylamide etc., metal hydrides such as sodium hydride, potassium hydride, phenyllithium, butyllithium etc., and amines, such as triethylamine, pyridine, α-, β- or γ- picoline, 2,6-lutidine, 4-dimethylaminopyridine, 4-(1-pyrrolidinyl)pyridine, dimethylaniline, diethylaniline etc. The reaction is carried out in an appropriate solvent at a temperature in the range of -20°C to the boiling point of the used solvent, preferably 0 to 150°C, for 1 min to 10 days. As the reaction solvent, there may be used, for example, liquid ammonia, alcohols (e.g., methanol, ethanol, propanol, isopropanol, butyl alcohol, sec-butyl alcohol, tert-butyl alcohol, ethylene glycol, methoxyethanol and ethoxyethanol), ethers (e.g., diemthyl ether, diethyl ether, tetrahydrofuran, dioxane, monoglyme adn diglyme), aliphatic hydrocarbons (e.g., pentane, hexane adn heptane), aromatic hdyrocarbons (e.g., benzene, toluene and xylene), dimethylformamide, dimethylsulfoxide, hexamethylphosphoramide, sulfolane and suitable solvent mixtures thereof.

Furthermore, Reformatsky reaction can be employed to carry out condensation. Referring to the reaction conditions for Reformatsky reaction, the reagent which is usable includes, for example, zinc, magnesium, aluminum and tin, and the reaction itself can be conducted in an appropriate solvent at a temperature in the range of -20°C to the boiling point of the used solvent, preferably 0 to 150°C, for 30 min to 3 days. As the solvent, there may be used, for example, ethers (e.g., dimethyl ether, diethyl ether, tetrahydrofuran, dioxane, monoglyme and diglyme), aliphatic hydrocarbons (e.g., pentane, hexane and heptane), aromatic hydrocarbons (e.g., benzene, toluene and xylene), and suitable solvent mixtures thereof.

The alkylation type reaction or amine exchange type reaction is carried out by allowing the Compounds (V) and (VI) to undergo reaction, as such or in an appropriate reaction solvent, at a temperature in the range of about -10°C to the boiling point of the used solvent, preferably about 10 to 80°C, for a period of time in the region of about 10 min to 48 hours. The reaction solvent includes, for example, water, alcohols (e.g., methanol, ethanol, propanol, isopropanol, butyl alcohol, sec-butyl alcohol, tert-butyl alcohol, ethylene glycol, methoxyethanol and ethoxyethanol), ethers (e.g., diethyl ether, tetrahydrofuran, dioxane, monoglyme and diglyme), halogenated hdyrocarbons (e.g., dichloromethane, chloroform and carbon tetrachloride), nitriles (e.g., acetonitrile), aliphatic hdyrocarbons (e.g., pentane, hexane and heptane), cyclic aliphatic hydrocarbons (e.g., cyclopentane and cyclohexane), aromatic hydrocarbons (e.g., benzene, toluene and xylene), nitromethane, pyridine, dimethylformamide, dimethylsulfoxide, hexamethylphosphoramide, sulfolane or suitable solvent mixtures thereof. Also, it in some instances is desirable to carry out the reaction in the presence of base, if necessary. The base which is usable includes, for example, the bases to be utilized in Wittig reaction. When a phase-transfer catalyst (e.g., cetyltrimetnylammonium chloride) is used at a ratio in the range of 0.01 to 0.2 equivalent, preferably 0.02 to 0.05 equivalent, against the Compound (V) or Compound (VI), furthermore, the reaction can be allowed to proceed advantageously, as well. In the case of the amine exchange type reaction, the reaction can in some instances be allowed to proceed under milder conditions, when the Compound (V) is converted into quaternary salts, such as salts with methyl bromide, methyl iodide, methyl methanesulfonate, methyl benzenesulfonate, methyl p-toluenesulfonate, etc. The above-mentioned reaction causing the Schiff base to be formed is conducted by allowing the Compound (V) and Compound (VI), as such or in an appropriate solvent, to undergo reaction at the molar ratio of (V)/(VI) = about 10 to 0.1 at a temperature in the range of -10°C to the boiling point of the used reaction solvent, preferably 0 to 50°C, for a period of time in the regiuon of about 10 min to 48 hours. In this reaction, the Compounds (V) and (VI), after having their aldehyde or ketone moieties protected in the form of acetal or ketal, may be used. As the reaction solvent, nonaqueous solvents are desirable, and there may be used, for example, alcohols (e.g., methanol, ethanol, propanol, isopropanol, butyl alcohol, sec-butyl alcohol, tert-butyl alcohol, ethylene glycol, methoxyethanol and ethoxyethanol), ethers (e.g., diemthyl ether, diethyl ether, tetrahydrofuran, dioxane, monoglyme and diglyme), esters (e.g., methyl acetate and ethyl acetate), halogenated hydrocarbons (e.g., dichloromethane, chloroform and carbon tetrachloride), nitriles (e.g., acetonitrile), aliphatic hydrocarbons (e.g., pentane, hexane, heptane and octane), cyclic aliphatic hdyrocarbons (e.g., cyclopentane and cyclohexane), aromatic hydrocarbons (e.g., benzene, toluene and xylene), acetone, nitromethane, pyridine, dimethylform- amide, dimethylsulfoxide, hexamethylphosphoramide, sulfolane and suitable solvent mixtures thereof. As the dehydrating agent, for example, molecular sieves, calcium chloride, magnesium sulfate, sodium sulfate and calcium sulfate can be added, or a pH value of the reaction solution can be adjusted suitably with acids (e.g., hydrochloric acid, hydrobromic acid, hydriodic acid, sulfuric acid, nitric acid and phosphoric acid), bases (e.g., metal hydroxides, such as sodium hydroxide, potassium hydroxide, lithium hydroxide and barium hydroxide ; sodium methoxide, sodium ethoxide, potassium tert-butoxide, sodium carbonate, potassium carbonate, barium carbonate, calcium carbonate, sodium hydrogencarbonate, trimethylamine, triethylamine, triethanolamine and pyridine) or buffers (e.g., phosphate buffers, borate buffers and acetate buffers) to thereby enhance and improve the reaction rate and yields. The reduction reaction and reductive alkylation reaction of the Schiff base are carried out through hydride reduction or catalytic reduction in an appropriate solvent at a reaction temperature in the range of about -40°C to the boiling point of the used solvent, more preferably about 0 to 50°C. As the solvent being usable, there are used the reaction sovlents to be utilized in the alkylation type reaction or amine exchange type reaction as described previously, as well as acetates (e.g., methyl acetate and ethyl acetate). As the catalytic reduction reaction, the previously mentioned Method C can be employed as such. The reagents which are used in the hydride reduction include, for example, lithium aluminum hydride, soidum borohydride, lithium borohydride and lithium cyanoborohydride, and the used amount of such reducing reagents is in the range of the equimolar to 100-fold molar quantity of the compound to be reduced, normally 2-fold to 20-fold the molar quantity.

When the ring A is furan, thiophene, thiophene-1-oxide, thiophene-1,1-dioxide or N-substituted pyrrole ring and $-Z^2-$ is -NH-, the said group -NH- in some instances undergoes ring-closure through condensation with the ring A to form a tricyclic compound (e.g., pyrrolo[3′,2′ :4,5]-pyrrolo[2,3-d]pyrimidine derivative). In such a case, the treatment with acids or bases can result easily in conversion into the objective bicyclic compound.

2nd Step :

The Compound (IV) as obtained in the 1st step can be convereted into the Compound (II) having its ester residual group [$-COOR^3$] to undergo a deesterification reaction as employed in the production of the Compound (I1). Also, the Compound (II) or (IV) can be partially reduced through a catalytic reduction reaction according to the Method C to thereby convert into the Compound (II′) or (IV′) which is understood to be included into the compounds of this invention.

The starting compound of the formula (II) where the ring A contains a heteroatom (nitrogen, oxygen and sulfur atoms) and $Q^1=Q^2=N$ can be produced for example by means of the reaction steps as shown in the following :

$$R^{10}-J^{1}-\overset{\overset{R'}{|}}{C}=\overset{\overset{R''}{|}}{C}-Z-B-COOR^{3} \qquad (VII)$$

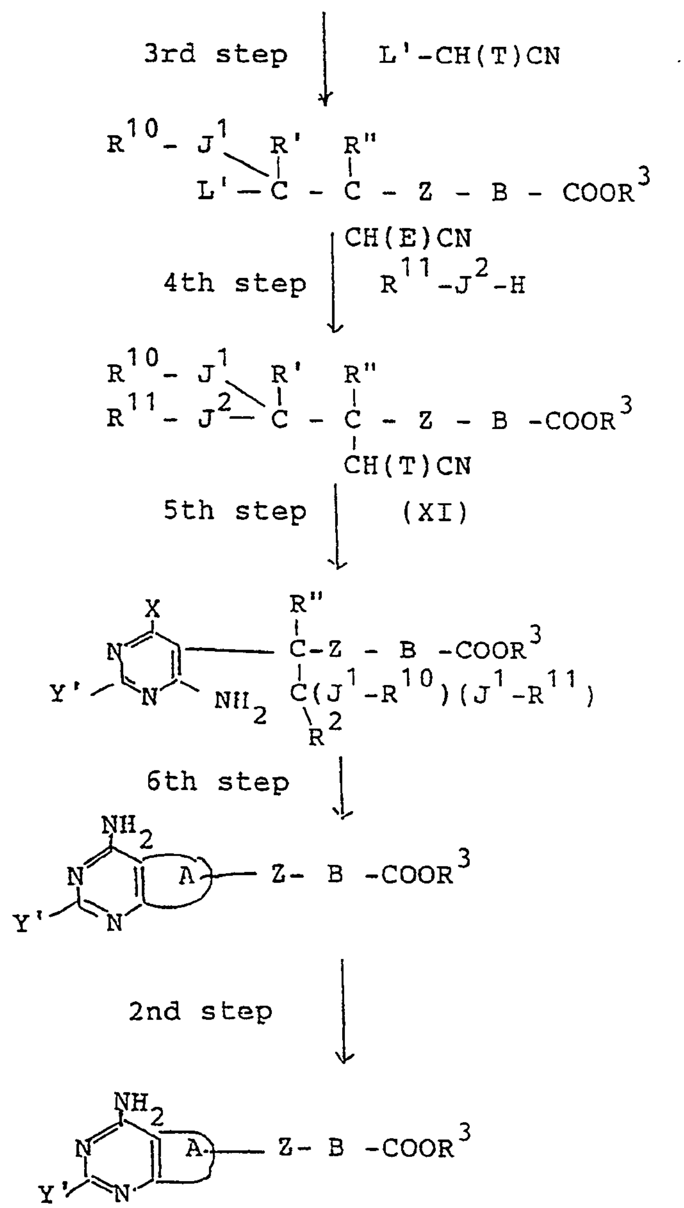


(VII)

(IX)

(X)

($IV_N$)

($II_N$)

In the above reaction steps, the ring A, B, $R^3$, X and Z are as defined hereinbefore ; $J^1$ and $J^2$ each is the same as or different from the other and represents oxygen or sulfur ; $R^7$ and $R^8$ each is the same as or different from the other and represents oxygen or sulfur ; $R^{10}$ and $R^{11}$ each is the same as or different from the other and represents a hdyrocarbon residue ; L' is a halogen atom (e.g., chlorine, bromine and iodine) ; T is a cyano group or a group represented by the formula $-COOR^{12}$, $-CSOR^{12}$ or $-CSSR^{12}$ ; Y', as defined or exemplified by Y, is a hydrogen atom, lower hydrocarbon residue, or aryl, 5- or 6-membered heterocyclic, hydroxyl, alkoxy, aryloxy, 5- or 6-membered heterocyclic-oxy, mercapto, alkylthio, arylthio, 5- or 6membered heterocyclic-thio, substituted amino, alkanoylamino, aroylamino or 5- or 6-membered heterocyclic-carbonylamino group ; R' and R' each is the same as or different from the other and represents a hdyrogen atom, or alkyl groups of $C_1$ to $C_3$, alkenyl groups of $C_2$ to $C_3$, alkynyl groups of $C_2$ to $C_3$ or cyclopropyl group as described in detail for the substituent on the ring A. Examples of the hydriocarbon residue represented by $R^{10}$, $R^{11}$ and $R^{12}$ include lower alkyl groups of $C_1$ to $C_5$ (e.g., methyl, ethyl, propyl, isopropyl, n-butyl, isobutyl, sec-butyl, tert-butyl, n-pentyl, iso-pentyl, sec-pentyl, neo-pentyl and tert-pentyl), benzyl and phenyl groups. These lower alkyl, benzyl and phenyl groups may have one to three substituents. Such substituents include, for example, halogen atoms (e.g., fluorine, chlorine, bromine and iodine), nitro group, cyano group, alkoxy groups of $C_1$ to $C_4$ (e.g., methoxy, ethoxy, propoxy, isopropoxy, n-butoxy, iso-butoxy, sec-butoxy and tert-butoxy groups), alkyl groups of $C_1$ to $C_4$ (e.g., methyl, ethyl, propyl, isopropyl, butyl, isobutyl, sec-butyl and tert-butyl groups), alkanoyl groups of

about $C_1$ to $C_4$ (e.g., formyl, acetyl, propionyl, n-butyryl and isobutyryl groups) and trifluoromethyl group.

Given below is detailed description on the above reaction steps :

3rd step :

This is a step where L'-CH(T)CN is allowed to undergo addition to the double bond ($R^{10}$-$J^1$-CH=CH-) in the compound (VII) to give the compound (VIII). The used amount of L'-CH(T)CN against the starting compound (VII) generally is about 0.5 to 4 mole equivalents, preferably about 0.8 to 1.5 mole equivalents. This reaction is carried out in an appropriate solvent at a reaction temperature in the range of about -10°C to the boiling point (up to about 100°C) of the used solvent, preferably about 0 to 50°C for about 30 min to 48 hours. As the solvent utilizable in the reaction, there are used, for example, alcohols (e.g., methanol and ethanol), ethers (e.g., dimethyl ether, diethyl ether, tetrahydrofuran, dioxane, monoglyme and diglyme), nitriles (e.g., acetonitrile), esters (e.g., ethyl acetate), halogenated hydrocarbons (e.g., dichloromethane, chloroform and carbon tetrachloride), aromatic hydrocarbons (e.g., benzene, toluene and xylene) and suitable solvent mixtures thereof. In conducting the reaction, irradiation with light or addition of organic peroxides can in some instances permit the reaction to proceed advantageously. The said organic peroxides inlcude, for example, t-butylhypochlorite, peracetic acid, perbenzoic acid and p-chloroperbenzoic acid. The compound (VIII) as obtained by the above procedure is relatively reactive and may be isolated in this stage, although it can also be used directly in the following step without being isolated.

4th step :

The compound (VIII) as obtained in the 7th step can be reacted with alcohols or thiols represented by $R^{11}$-$J^2$-H in an appropriate solvent at a reaction temperature in the range of about -10°C to the boiling point (up to about 100°C) of the used solvent, preferably about 0 to 50°C, for about 10 min to 24 hours to thereby produce the compound (X). As the solvent being utilizable in the reaction, there may be used, for example, ethers (e.g., dimethyl ether, diethyl ether, tetrahydrofuran, dioxane, monoglyme and diglyme), nitriles (e.g., acetonitrile), esters (e.g., ethyl acetate), halogenated hdyrocarbons (e.g., dichloromethane, chlorform and carbon tetrachloride), aromatic hydrocarbons (e.g., benzene, toluene and xylene) or suitable solvent mixtures thereof. The alcohols or thiols represented by $R^{11}$-$J^2$-H may be used in excess to utilize as a solvent.

5th step :

The compound (IX), upon treatment with a compound represented by the general formula

Y'-C=NH      (XI)
    \
     $NH_2$

[wherein Y' is as defined hereinbefore] or its salt, can allow reaction through its cyano, ester or thioester group, and undergo cyclization, while it forms the pyrimidine ring, to thereby produce the Compound (X). The acid salt of the Compound (XI) includes, for example, salts formed with minerla acids, such as hydrochloric acid, hydrobromic acid, hydriodic acid, sulfuric acid, nitric acid, phosphoric acid and boric acid, and with organic acids, such as oxalic acid, tartaric acid, lactic acid, citric acid, acetic acid, trifluoroacetic acid, methanesulfonic acid, ethanesulfonic acid, benzenesulfonic acid, p-toluenesulfonic acid and camphorsulfonic acid, while the base salt of the compound (XI-1 ; Y'= hydroxyl or mercapto group) includes, for example, salts formed with sodium, potassium, lithium, calcium, magnesium, aluminum, zinc, ammonium, trimethylammonium, triethylammonium, triethanolammonium, pyridinium, substituted pyridinium, etc.

On the occasion of ring-closure, the reaction, when conducted under basic conditions, can in some instances be allowed to proceed advantageously. The base being usable includes, for example, metal alkoxides, such as sodium methoxide, sodium ethoxide and potassium tert-butoxide. The reaction temperature ranges from 0 to 150°C, preferably from 20 to 100°C, while the rection time is 1 to 48 hours. As the reaction solvent, there may be used, for example, methanol, ethanol, propanol, tert-butyl alcohol, dimethylsulfoxide, hexamethylphosphoramide or suitable solvent mixtures thereof.

6th step :

If necessary, the group HC($J^1$-$R^7$)($J^1$-$R^8$) in the Compound (X) can be restored to a carbonyl group, bringing about spotaneously an intramolecular ring-closure reaction, to convert the Compound (X) into the Compound ($IV_N$). The restoration reaction to a carbonyl group can be carried out by subjecting the compound (X), as such or in a suitable reaction solvent, to a decomposition raection at a reaction temperature in the range of about -10°C to the boiling point (up to about 100°C) of the used solvent, preferably about 0 to 50°C, for a period of time in the region of about 10 min to 100 hours. The said decomposition reaction includes, for example, a hydrolysis reaction under acidic conditions (Method B-1), decomposition reaction under acidic, non-aqueous conditions (Method B-2), catalytic reduction reaction (Method C), decomposition reaction with use of metal salts (Method D) or decomposition reaction with oxidizing agents (Method E). The Methods B1, B-2 and C can be

carried out by following, as such, the procedures as illustrated in detail for the decomposition reactions of the groups represented by the formulae $-COOR^1$ and $-COOR^2$. The metal salts which are usable in the Method D include, for example, cupric chloride, silver nitrate, silver oxide, mercuric chloride and tellurium salts (e.g., tellurium nitrate and tellurium trifluoroacetate), while the oxidizing agents which are utilizable in the Method E include, for example, oxygen-light, hydrogen peroxide, perbenzoic acid, m-chloroperbenzoic acid, perchlorates (e.g., lithium perchlorate, silver perchlorate, mercuric perchlorate and tetrabutylammonium perchlorate), nitrosylsulfuric acid, alkyl nitrites (e.g., isoamyl nitrite), iodine, bromine, chlorine, N-bromosuccinimide, sulfuryl chloride and chloramine T. Which method should be applied to restore a carbonyl group ($>$C=O) can be suitably decided depnding upon the chemcial properties of $-J^1-R^{10}$ and $-J^2-R^{11}$. As the reaction solvent, there may be used, for example, water, alcohols (e.g., methanol, ethanol, propanol, isopropanol, butyl alcohol, sec-butyl alcohol, tert-butyl alcohol, ethylene glycol, methoxyethanol and ethoxyethanol), ethers (e.g., dimethyl ether, diethyl ether, tetrahydrofuran, dioxane, monoglyme and diglyme), aromatic hydrocarbons (e.g., benzene, toluene and xylene), halogenated hydrocarbons (e.g., dichloromethane, chloroform and carbon tetrahchloride), acetone, acetonitrile or suitable solvent mixtures thereof. dichloromethane, chloroform and carbon tetrahchloride), acetone, acetonitrile or suitable solvent mixtures thereof. The intramolecular ring-closure and dehydration reactions in the step of producing the compound ($IV_N$) normally allow the group X on the pyrimidine ring to condense spontaneously to the carbonyl group ($>$C=O) in the course of or after restoration to thereby form the ring A. In conducting the reaction, the presence of acid catalysts can also permit the said reaction to proceed quickly and in improved yields. The said acid catalysts include, for example, mineral acids, organic acids and Lewis acids as described in detail for the Methods B-1 and B-2. Also, the carbonyl group ($>$C=O) can be reudced to a hydroxymethyl group ($-CH_2OH$), which hydroxymethyl moiety can then be converted into the removal group L, followed by alkylation reaction with the group X in the same molecule to thereby produce the compound ($IV_{N'}$) having the ring A reduced partially. The carbonyl-group reduction reaction and the conversion reaction of the hydroxyl group into the removal group as well as the intramolecular alkylation reaction are carried out by employing the per se known procedures. In addition, the compound ($II_N$) or ($IV_N$) can be subjected to a catalytic reduction reaction according to the Method C to perform partial reduction, thus being converted into the compound ($II_{N'}$) or ($IV_{N'}$) wherein the ring A is partially hydrogenated.

In the case of the compound ($IV_N$) or ($IV_{N'}$) where the ring A is a pyrrole or pyrroline ring, the compound (X), ($IV_N$) or ($IV_{N'}$) can be subjected to the per se known alkylation or acylation reaction to be converted into the compound having an N-substituted pyrrole or N-substituted pyrroline ring, which falls into the scope of this invention. Furthermore, the above-mentioned alkylation reaction or acylation reaction can be carried out with use of the known compounds [The Japanese Unexamined Patent Publicaiton No. 72235/1989] to produce the compounds of this invention containing an N-substituted pyrrole or N-substituted pyrroline ring as the ring A.

The starting compound ($II_c$) of the formula (II) where the ring A consist solely of carbon atoms $Q^1=Q^2=N$ can be produced, for example, by means of the reaction steps as shown in the following :

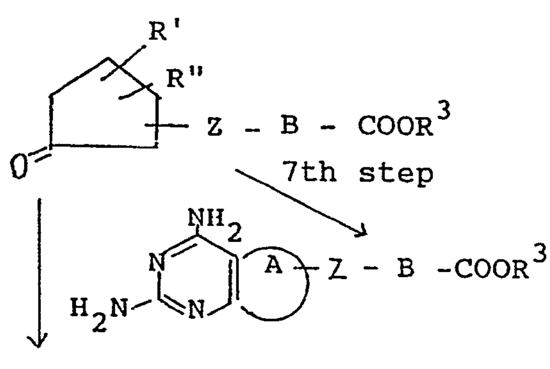


(XII)

($IV_{C1}$)

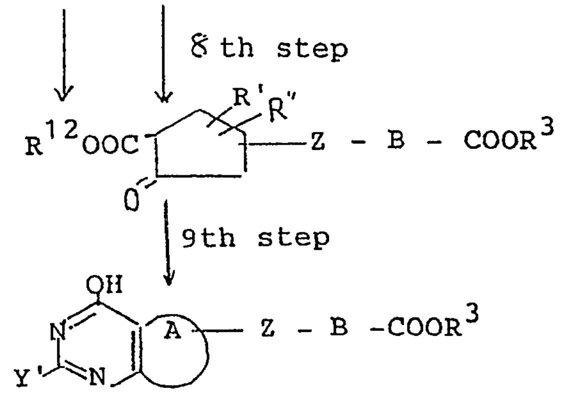


(XIII)

($IV_{c2}$)

In the above reaction steps, the ring A, B, $R^3$, $R^{12}$,R′, R″, X andd Y′ are as defined hereinbefore, and R′, R″ and -Z- B -$COOR^3$ are to be understood to form bonding in the consecutive three positionsion on the cyclopentane ring.

7th step :

The Compound (XII) as synthesized by the conventional procedure, upon treatment under heating with dicyandiamide, undergoes a cyclization reaction to form a condensed pyrimidine ring, thus yielding the Compound ($IV_{C1}$), whereby the reaction temperature suitably ranges from 100 to 300°C, more preferably from 150 to 250°C, with the reaction time being 1 to 24 hours. If necessary, furtehrmore, dehydrogenation can be effected by following the per se known procedure with the known reagents to introduce an unsaturated bond into the ring A.

8th step :

Out of the two alpha-positions adjacent to the carbonyl group in the Compound (XII), the hydrogen at the alpha- position not being substituted with R′, R″ or -Z- B -$COOR^3$ is drawn in accordance with the conventional method to form a carbanion, and an ester residue is introduced into the activated position to thereby produce the Compound (XIII).

9th step :

The Compound (XIII), upon treatment with the compound of the general formula (XI), allows the reaction through its carbonyl group and ester residue, bringing about ring-closure and cyclization, whereupon a condensed pyrimidine ring is formed anew to produce the Compound ($IV_{C2}$). With reference to the reaction conditions, the conditions as employed in the fifth step can be applied as such. If necessary, furthermore, dehydrogenation can be performed in accordance with the per se known method with use of the known reagents to introduce an unsaturated bond into the ring A.

The ester derivatives ($IV_{C1}$) and ($IV_{C2}$), as obtained in the 7th and 9th reaction steps, can be subjected to the deesterification reaction in the same manner as described in the 2nd step to tehreby be converted into their corresponding carboxylic acid derivatives ($II_{C1}$) and ($II_{C2}$), respectively.

In cases where B is a cycloalkenylene group or a substituted phenylene group, such groups may be subjected to a catalytic reduction reaction in either suitable step of the 1st step through the 9th step to thereby be converted into the corresponding cycloalkylene group. As the said catalytic reduction reaction, the previously mentioned Method C as such is advantageneously applied.

When Y′ is a hydroxyl, alkoxy, aryloxy, 5- or 6-membered heterocyclic-oxy, mercapto, alkylthio, arylthio, 5- or 6-membered heterocyclic-thio, arylthio, 5- or 6-membered heterocyclic-thio, substituted amino, alkanoylamino, aroylamino or 5- or 6-membered heterocyclic-carbonylamino group, such groups may be subejcted to the per se known conversion reaction in either suitable step of the 2nd, 6th, 7th and 9th steps to be convereted into the 5- or 6membered heterocyclic group, halogen atom, or carboxyl, carbamoyl, nitro, hydroxyl, alkoxy, aryloxy, 5- or 6-membered heterocyclic-oxy, mercapto, alkylthio, arylthio, 5- or 6membered heterocyclic-thio, substituted amino, alkanoylamino, aroylamino, 5- or 6-membered heterocyclic-carbonylamino, alkanoyloxy, aroyloxy, or 5- or 6-membered carbonyloxy group, as exemplified by Y.

In cases in which the ring A and B contains a sulfur atom or -$Z^2$- is -S- (sulfur atom), the Compound (I) of this invention can be subjected, either directly or in either step of the feasible aribitrary steps, to an oxidation reaction to be converted into the compound where the sulfur atom in the ring A, B and -$Z^2$- is changed into $S(O)_n$ [n=1 or 2]. The oxidation reaction can be carried out in appropriate solvent, normally in the presence of an oxidizing agent of 0.3 to 3.0 equivalents against the compound to be oxidized, preferably 0.5 to 2.5 equivalents, at a temperature of -10 to +100°C, preferably 0 to 50°C, for 10 min to 48 hours, desirably 30 min to 24 hours to produce the said compound. Preferred examples of the oxidizing agent which is to be used in the reaction

include peroxides (e.g., hydrogen peroxide, peracetic acid, perbenzoic acid and m-chloroperbenzoic acid). As the reaction solvent, there are used, for example, water, acetic acid, ketones (e.g., acetone and methyl ethyl ketone), ethers (e.g., dimethyl ether, diethyl ether, dioxane, monoglyme and diglyme), halogenated hydrocarbons (e.g., dichloro- methane, chloroform and carbon tetrachloride), aliphatic hydrocarbons (e.g., pentane, hexane, heptane and octane), cyclic aliphatic hydrocarbons (e.g., cyclopentane and cyclohexane), aromatic hydrocarbons (e.g., benzene, toluene and xylene) or suitable solvent mixtures thereof.

Furthermore, the amino, hydroxyl or mercapto group as represented by X in the Compounds (I), (II) and (IV) can be converted into one another, as the case may be, by means of substituent replacement reactions on the pyrimidine ring known in literature [supplement volume of "Tanpakushitsu/Kakusan/Kouso (Proteins/Nucleic Acids/ Enzymes)", Chemical Synthesis of Nucleic Acid, Published by Kyoritsu Publishing Co. of Japan (1968)].

In addition, the reactions, reagents and reaction conditions as well as the protective groups to be applied for functional groups as used if necessary, which are to be carried out or employed in the 1st step through 9th step or in the step of producing the starting compound, are already known and described in detail in the following literature [J. F. W. McOmine, "Protective Groups in Organic Chemistry", Plenum Press, London and New York (1973)], [Pine, Hendrickson and Hammond, "Organic Chemistry" (4th edition), [I] to [II], Hirokawa Shoten of Japan] and [M. Fieser and L. Fieser, "Reagents for Organic Synthesis", vol. 1 to 13, Wiley Interscience, New York, London, Sydney and Tronto (1969-1988)].

The each intermediate of the present compounds as well as the present compounds (I) and their salts can be isolated from the reaction mixture by usual separating means, for example, concentration, extraction with a solvent, chromatography, recrystallization, etc.

The Compounds (I), (II) and (IV) obtainable by the production method of this invention may form salts. Examples of the salts with bases include salts formed with alkali metals, alkaline earth metals, non-toxic metals, ammonium and substituted ammoniums, such as sodium, potassium, lithium, calcium, magnesium, aluminum, zinc, ammonium, trimethylammonium, triethylammonium, triethanolammonium, pyridinium and substituted pyridinium. The salts with acids include, for example, salts formed with mineral acids, such as hydrochloric acid, sulfuric acid, nitric acid, phosphoric acid and boric acid, and salts formed with organic acids, such as oxalic acid, tartaric acid, acetic acid, trifluro- acetic acid, methanesulfonic acid, benzenesulfonic acid, p-toluenesulfonic acid and camphorsulfonic acid.

The Compounds (I) of this invention and their salts exhibit inhibitory activity against not less than one kind of the enzymes utilizing folic acid and its related compounds as a substrate. Consequently, these compounds can be used either alone or in combination with other antitumor agents for the purpose of treatment of choriocarcinoma, leukemia, breast adenocarcinoma, head and neck cancer (epithelioma), squamocarcinoma, cellule lung cancer and lymphosarcoma that have been treated so far with MXT, as well as other various tumors.

The Compounds (I) or their salts, when intended to be used as an atitumor agent, can be administered orally or parenterally, as such or after being processed into such dosage forms as powder, granule, tablet, capsule, suppository and injectable solution by means of the conventional procedures with use of pharmacologically allowable carriers, excipients, diluents, etc. Their dosage amount varies depending upon the species of subject animals (warm-blooded animals such as humans, dogs, cats, rabbits, monkeys, rats, mice, etc.), type of diseases, severity of symptoms, kind of compounds, route of administration, etc., and their daily dose for the above warm-blooded animals is about 2.0 to 200 mg/kg body weight, preferably 4.0 to 80 mg/kg body weight, as the compound of this invention in the case of oral administration, while the daily dose ranges from about 1.0 to 100 mg/kg body weight, preferably 2.0 to 40 mg/kg body weight, in the case of parenteral administration. The method of administration for injectable solutions includes, for example, intramuscular injection, intraperitoneal injection, subcutaneous injection and intravenous injection.

The above mentioned procedures of processing into pharmaceutical preparations are conducted into practice in accordance with the per se known methods. In manufacturing the above-mentioned oral preparations, for example, tablets, binders (e.g., hydroxypropylcellulose, hydroxypropyl methylcellulose and macrogoal), disintegrating agents (e.g., starch and carboxymethylcellulose calcium), lubricants (e.g., magnesium stearate and talc) and others can suitably be formulated.

In producing such non-oral, parenteral pharmaceutical preparations as injectable solution, tonicity agents (e.g., glucose, D-sorbitol, D-mannitol and sodiumchloride), preservatives (e.g., benzyl alcohol, chlorobutanol, methyl p-oxybenzoate and propyl p-oxybenzoate), buffering agents (e.g., phosphate buffers and sodium acetate buffers) and others can be suitably incorporated.

With reference to a specific example of the manufacture of tablets, for example, about 1.0 to 50 mg of the present compound (I) or its salt, 100 to 500 mg of lactose, about 50 to 100 mg of corn starch and about 5 to 20 mg of hydroxypropylcellulose, being weighed out for use in the manufacture of one tablet, are mixed, granulated and admixed with corn starch and magnesium stearate, followed by compression into a tablet weighing

about about 100 to 500 mg and measuring about 3 to 10 mm in diameter, in accordance with the conventional procedures. The resulting tablet can furthermore be processed into an enteric-coated tablet by providing coating with use of a ca. 5 to 10 % solution of hydroxypropylmethylcellulose phthalate (about 10 to 20 mg per tablet) and castor oil (about 0.5 to 2.0 mg per tablet) in acetone-ethanol mixture.

Referrring to a specific example of producing injectable solutions, for example, about 2.0 to 50 mg per ampoule of sodium salt of the present compound (I), (i) is dissolved in about 2 ml of isotonic saline, and the solution is filled into an ampoule, followed by fusion and heat sterilization at about 110°C for about 30 min, or (ii) is dissolved in a solution of about 10 to 40 mg of mannitol or sorbitol in about 2 ml of sterilized distilled water is filled into an ampoule, followed by lyophilization and fusion to thereby prepare injectable solution. On the occasionof the use of the lyophilized compound, the said ampoule is opened, and, for example, physiological saline is poured therein to make a solution having a concentration of about 1.0 to 25 mg/ml of the compound. The solution can be employed as an injectable preparation in the subcutaneous, intravenous or intramuscular administration.

Described below are the reference examples and examples to illustrate this invention specifically.

Reference Example 1

Production of methyl 4-[2-(2-amino-7-benzyl-3-isopropyloxymethyl-4(3H)-oxopyrrolo[2,3-d]pyrimidine-5-yl)-ethyl]benzoate :

Methyl 4-[2-(2-mino-7-benzyl-3-isopropyloxymethyl-4(3H)-oxopyrrolo-[2,3-d]pyrimidine-5-yl)ethenyl benzoate (4.36 g) was dissolved in a mixed solution of methanol-tetrahydrofuran (1 :1, 400 ml), and after addition of a 50 % aqueous solution of 10 % Pd-C (4.36 g, manufactured by Engelhardt Co.), catalytic reduction was performed under an atmosphere of hydrogen for 30 min. The catalyst was filtered out, and the filtrate was concentrated under reduced pressure to give the subject compound (4.03 g).

IR (Neat) : 3320, 2980, 1720, 1680, 1620, 1540, 1280, 1110, 1060 $cm^{-1}$.

$^1$H-NMR ($CDCl_3$) δ : 1.20(6H,d), 3.03(4H,s), 3.87(3H,s), 3.73-3.97(1H,m), 5.05(2H,s), 5.60(2H,s), 6.10(1H,s), 7.00-7.40 (7H,m), 7.88(2H,d).

Reference Example 2

Production of methyl 4-[2-(2-amino-7-benzyl-6-bromo3-isopropyloxymethyl-4(3H)-oxopyrrolo[2,3-d]-pyrimidine-5-yl)ethyl]benzoate :

The compound (4.00 g) of Reference Example 1 was dissolved in dried dichloromethane (188 ml), and N-bromosuccinimide (1.68 g) was added to the solution under stirring with ice-cooling, followed by standing at the same temperature for 30 min. The solvent was distilled off under reduced pressure, and the residue was separated and purified by silica gel column chromatography [carrier : 100 g, developing solvent ; hexane ;ethyl acetate = 7 :3] to give the subject compound (4.74 g).

$^1$H-NMR ($CDCl_3$) δ: 1.20(6H,d), 3.03(4H,s), 3.87(3H,s), 3.70-4.00(1H,m), 5.18(2H,s), 5.57(2H,s), 7.00-7.33(7H,m), 7.88 (2H,d)

Example 1

Productionof methyl 4-[2-(2-amino-7-benzyl-6-bromo4-hydroxy-7H-pyrrolo[2,3-d]pyrimidine-5-yl)ethyl]-benzoate :

The compound (4.51 g) of Reference Example 2 was dissolved in a dichloromethane solution (407 ml) of 0.21 N hydrogen bromide, followed by stirring at room temperature for 17 hours. Hexane (800 ml x 2) was added to the reaction liquid, and the supernatant solution was removed. The residue was separated and purified by silica gel column chromatography [carrier ; 500 g, developing solvent ; hexane : ethyl acetate = 3 :2→ ethanol :chloroform = 8 :92] to give the subject compound (2.93 g).

$^1$H-NMR (DMSO-$d_6$) δ : 2.93(4H,bs), 3.87(3H,s), 5.13(2H,s), 6.87-7.07(2H,m), 7.13-7.33(5H,m), 7.82(2H,d).

Example 2

Production of 4-[2-(2-Amino-7-benzyl-6-bromo-4-hydroxy-7H-pyrrolo[2,3-d]pyrimidine-5-yl)ethyl]benzoic acid :

The compound (306 mg) of Reference Example 1 was dissolved in a mixed solution of 0.2N aqueous sodium hdyroxide solution (15.9 ml) and tetrahydrofuran (8 ml), followed by standing at room tremperature for 18 hours under stirring. Major poriton of the tetrahydrofuran was distilled off, and the residual solution was neutralized by adding 0.2N hydrochloric acid (15.9 ml) under stirring with ice cooling. The resulting precipitate was recovered by filtration, washed with water, methanol and ether successively, and dried to give the subject compound (268 mg).
IR (Nujol) : 3470, 3130, 1710, 1680, 1650, 1605, 1460, 1380, 1260, 1170 $cm^{-1}$.
$^1$H-NMR (DMSO-$d_6$) δ : 2.92(4H,s), 5.13(2H,s), 6.27(2H,s), 6.87-7.07(2H,m), 7.13-7.37(5H,m), 7.82(2H,d).

Example 3

Production of diethyl N-[4-[2-(2-amino-7-benzyl-6-bromo-4-hydroxy-7H-pyrrolo[2,3-d]pyrimidine-5-yl)ethyl]-benzoyl]-L-glutamate :

The compound (234 mg) of Reference Example 2 and diethyl glutamate hdyrochloride (240 mg) were dissolved in dried dimethylformamide (15 ml), and the solution was cooled to 0°C and then admixed with diphenylphosphoryl azide (162 ul) and triethylamine (280 ul), followed by stirring at the same temperature for 2 hours and then at room temperature for 5 hours. The resulting precipitate was filtered out, and after the filtrate was concentrated to dryness, the residue was separated andpurified by silica gel column chromatography [carrier; 75 g, developing solvent ; hexane :ethyl acetate = 3 :7→ethyl acetate] to give the subject compound (229 mg).
$^1$H-NMR ($CDCl_3$) δ : 1.20(3H,t), 1.27(3H,t), 2.00-2.63(4H,m), 3.00(4H,s), 3.97-4.33(4H,qx2), 4.63-4.93(1H,m), 5.17(2H,s), 7.00-7.43(7H,m), 7.70(2H,d).

Example 4

Production of diethyl N-[4-[2-(2-amino-7-benzyl-4-hydroxy-7H-pyrrolo[2,3-d]pyrimidine-5-yl)ethyl]benzoyl]-L-glutamate :

The compound (234 mg) of Example 3 was dissolved in a mixed solution (2 :1, 15 ml) of ethanol-tetrahydrofuran, and after addition of 1N hydrochloric acid (0.66 ml) and a 50 % aqueosu olution of 10 % Pd-C (862 mg, manufctured by Engelhardt Co.), catalytic reduction was carried out under an atmosphere of hdyrogen at 4 atmospheric pressure for 5 hours. The catalyst was filtrered out, and the filtrate was neutralized with 1N aqueous sodium hydroxide solution and concentrated to dryness underreduced pressure. The residue was separated andpurified by silica gel column chromatography (carrier ; 45 g, developing solvent ; ethanol : chloroform = 4 :96→15 :85] to give the subject compound (135 mg).
$^1$H-NMR ($CDCl_3$) δ : 1.20(3H,t), 1.29(3H,t), 1.87-2.60(4H,m), 3.00(4H,s), 3.97-4.35(4H,qx2), 4.63-4.93(1H,m), 5.07(2H,s), 5.24(2H,s), 6.14(1H,s), 7.05(2H,d), 7.10-7.37(5H,m), 7.68-7.70(2H,d).

Example 5

Production of N-[4-[2-(2-amino-7-benzyl-6-bromo-4-hydroxy-7H-pyrrolo[2,3-d]pyrimidine-5-yl)ethyl]benzoyl]-L-glutamic acid :

The compound (160 mg) of Example 3 was dissolved in a mixed solution (1 :2, 6 ml) of water-tetrahydrofuran, and 1N aqueous sodium hydroxide solution (0.736 ml) was added to the solution, followed by stirring at room temperature for 1 hour. The reaction solution was concentrated to the solvent volume of 2 ml under reduced pressure, and the resulting insoluble matter was filtered out through Millipore, followed by cooling at 0°C of the filtrate and addition of acetic acid (0.2 ml). The resulting precipitate was recovered by filtration, washed with ice water and dried under reduced pressure to give the subject compound (128 mg).
$^1$H-NMR (DMSO-$d_6$) δ : 1.82-2.45(4H,m), 2.91(4H,s), 4.15-4.46 (1H,m), 5.12(2H,s), 7.05-7.42(7H,m), 7.73(2H,d).

Example 6

Production of N-[4-[2-(2-amino-7-benzyl-4-hydroxy-7H-pyrrolo[2,3-d]pyrimidine-5-yl)ethyl]benzoyl]-L-glutamic acid :

By following the same procedure as described in Example 5, the subject compound (115 mg) was produced from the compound (160 mg) of example 4.
$^1$H-NMR (DMSO-$d_6$) δ : 1.79-2.50(4H,m), 2.93(4H,s), 4.18-4.50(1H,m), 5.03(2H,s), 6.11(1H,s), 7.06(2H,d), 7.12-7.39(5H,m), 7.69(2H,d).

Reference Example 3

Production of 4-amino-5-formyl-7-methylpyrrolo[2,3-d]pyrimidine :

Raney-nickel (5.0 g) was added to a solution (25 ml) of 4-amino-5-cyano-7-methylpyrrolo[2,3-d]pyrimidine (600 mg) in formic acid, and then the mixture was stirred at 75 to 80° C for 1.5 hours. The reaction mixture was filtered when it was hot, and the residue was washed with formic acid until the washings became colorless. The filtrate and washings were combined and concentrated under reduced pressure and the resulting residue was dissolved in hot water. The solution was adjusted to pH 7.0 with 1 N aqueous sodium hydroxide solution and extracted with chloroform. The extract solution was dried with anhydrous magnesium sulfate and the solvent was removed from the solution under reduced pressure to give the subject compound as colorless powder (370 mg).

$^1$H-NMR (DMSO-$d_6$) δ : 3.78(3H,s), 7.57(2H,m), 8.21(1H,s), 8.27(1H,s), 9.67(1H,s)

IR (KBr) : 3350, 3100, 1650, 1595 $cm^{-1}$

Reference Example 4

Production of methyl 4-[2-(4-amino-7-methylpyrrolo[2,3-d]pyrimidine-5-yl)ethenyl]benzoate :

Under argon atmosphere, a methanol solution (0.2 ml) of 4.9 moles of sodium methoxide was added to a solution (10 ml) of p-carbomethoxybenzyltriphenylphosphonium bromide (500 mg) in dried N,N-dimethylformamide, and the mixture was stirred at room temperature for 15 minutes. To the resultant solution, a solution (10 ml) of the compound (180 mg) of Reference Example 3 in dried N,N-dimethylformamide was added, the mixture was stirred at room temperature for further 3 days, and solvent was removed from the resultant mixture under reduced pressure. The residue thus obtained was purified by silica gel column chromatography [carrier: 60 g, developing solvent : chloroform/methanol = 19/1] to give the subject compound (200 mg).

$^1$H-NMR ($CDCl_3$) δ: 3.83(3H,s), 3.93(3H,s), 5.46(2H,m), 6.70-6.94(1H,m), 7.16(1H,s), 7.29-7.39(1H,m), 7.53(2H,d,j=8.4Hz), 7.87(2H,d,j=8.4Hz), 8.35(1H,s)

Example 7

Production of diethyl N-[4-[2-(2-amino-4-hydroxy-7-methyl-7H-pyrrolo[2,3-d]pyrimidine-5-yl-)ethyl]benzoyl]-L-glutamate :

Starting from methyl 4-[2-(2-amino-7-methyl-3-isopropyloxymethyl-4(3H)-oxopyrrolo[2,3-d]pyrimidine-5-yl)ethenyl]benzoate, methyl 4-[2-(2-amino-6-bromo-4-hydroxy-7-methyl-7H-pyrrolo[2,3-d]pyrimidine-5-yl)ethyl]-benzoate was obtained in the same manners as in Reference Examples 1 and 2 and Example 1. The resulting compound (4.06 g) was subjected to the same procedures as in Examples 2, 3, 4 and 6 in order, whereby the subject compound (3.03 g) was obtained.

$^1$H-NMR ($CDCl_3$/$CD_3OD$) δ: 1.22(3H,t,j=7.2Hz),1.30(3H,t,j=7.2Hz), 2.07-2.39(2H,m), 2.44-2.57(2H,m), 3.03(4H,s), 3.57(3H,s), 4.11(2H,q,j=7.2Hz), 4.24(2H,q,j=7.2Hz), 4.73-4.86(1H,m), 5.31(2H,s), 6.21(1H,s), 7.25(2H,d,j=8.2Hz), 7.32(1H,d,j=7.4Hz), 7.70(2H,d,j=8.2Hz)

Example 8

Production of N-[4-[2-(2-amino-4-hydroxy-7-methyl-7H-pyrrolo[2,3-d]pyrimidine-5-yl)ethyl]benzoyl]-L-glutamic acid :

The compound (0.995 g) of Example 7 was subjected to hydrolysis reaction in the same manner as in Example 5, thereby the subject compound (0.768 g) was obtained.

$^1$H-NMR ($Me_2SO$-$d_6$) δ : 1.84-2.08(2H,m), 2.30(2H,t,j=7.4Hz), 2.79-3.04(4H,m), 3.55(3H,s), 4.31-4.43(1H,m), 6.12(2H,s), 6.39(1H,s), 7.27(2H,d,j=8.2Hz), 7.75(2H,d,j=8.2Hz), 8.41(12H,d,j=7.4Hz), 10.25(1H,s)

Example 9

Production of diethyl N-[4-[2-(2-amino-4-hydroxy-7-methyl-6,7-dihydro-5H-pyrrolo[2,3-d]pyrimidine-5-yl)ethyl]benzoyl]-L-glutamate :

The subject compound (1.99 g) of Example 7 was dissolved in trifluoroacetic acid (40 ml), and 10 % Pd-C containing 50 % of water (1.0 g) was added thereto. The mixture was stirred for 48 hours under hydrogen atmosphere, the catalyst was removed therefrom by filtration, and the filtrate was neutralized and concentrated under reduced pressure. The resultant residue was purified by silica gel chromatography (carrier : 140 g, developing solvent : chloroform : methanol = 1 :39--1 :9) to give the subject compound (0.92 g).

$^1$H-NMR ($Me_2SO$-$d_6$) δ: 1.16(3H,t,j=7.2Hz),1.20(3H,t,j=7.2Hz), 1.48-1.69(1H,m), 1.93-2.18(3H,m), 2.46(2H,t,j=7.4Hz), 2.66(2H,t,j=7.2Hz), 3.02-3.17(2H,m), 3.43-3.56(1H,m), 3.58(3H,s), 4.05(2H,q,j=7.2Hz), 4.11(2H,q,j=7.2Hz), 4.38-4.50(1H,m), 6.15(2H,s), 7.30(2H,d,j=8.2Hz), 7.78(2H,d,j=8.2Hz),

8.64(1H,d,j=7.4Hz), 9.54(1H,s)

Example 10

Production of N-[4-[2-(2-amino-4-hydroxy-7-methyl-6,7-dihydro-5H-pyrrolo[2,3-d]pyrimidine-5-yl)ethyl] benzoyl]-L-glutamic acid :

The compound (0.85 g) of Example 9 was subjected to hydrolysis reaction in the same manner as in Example 5, thereby the subject compound (0.64 g) was obtained.

$^1$H-NMR ($Me_2SO$-$d_6$) : 1.53-1.73(1H,m), 1.87-2.21(3H,m), 2.37(2H,t,j=7.8Hz), 2.68(2H,t,j=7.2Hz), 3.02-3.19(2H,m), 3.41-3.55(1H,m), 3.56(3H,s), 4.37-4.49(1H,m), 6.17(2H,s), 7.30(2H,d,j=8.4Hz), 7.79(2H,d,j=8.4Hz), 8.50(1H,d,j=7.8Hz)

Example 11

Production of 4-[3-(2-amino-4-hydroxy-7-methyl-7H-pyrrolo[2,3-d]pyrimidine-5-yl)propyl]benzoic acid :

Methyl 4-[3-(2-amino-3-isopropyloxymethyl-7-methyl-4(3H)oxopyrrolo[2,3-d]pyrimidine-5-yl)-1-oxo-2-propenyl]benzoate (6.37 g)was dissolved in a mixed solution (300 ml) of methanol-tetrahydrofuran (1 :1), 10 % Pd-C containing 50 5 of water (6.4 g, produced by Engelhardt Co.) was added thereto, and the mixture was stirred vigorously for 30 hours under hydrogen atmosphere. The catalyst was removed from the reaction mixture by filtration and the filtrate was concentrated under reduced pressure to give crude methyl 4-[3-(2-amino-3-isopropyloxy-7-methyl-4(3H)-oxopyrrolo[2,3-d]pyrimidine-5-yl)propyl]benzoate. The total amount of the crude product was subjected to the same reaction removing isopropyl group as in Example 1 and then to the same hydrolysis reaction as in Example 2, thereby the subject compound (1.98 g) was obtained.

$^1$h-NMR ($Me_2SO$-$d_6$) δ: 1.80-2.18(2H,m), 2.51-2.82(2H,m), 3.57(3H,s), 6.03(2H,s), 6.41(1H,s), 7.26(2H,d,j=8Hz), 7.77(2H,d,j=8Hz)

Example 12

Production of diethyl N-[4-[3--(2-amino-4-hydroxy-7-methyl-7H-pyrrolo[2,3-d]pyrimidine-5-yl)propyl]benzoyl]-L-glutamate :

In the same manner as in Example 3, the compound (1.74 g) was obtained from the subject compound (1.63 g) of Example 11.

$^1$H-NMR ($CDCl_3$/$CD_3OD$) δ : 1.21(3H,t,j=7.2Hz), 1.27(3H,t,j=7.2Hz), 1.87-2.35(4H,m), 2.37-2.57(2H,m), 2.61-2.93(4H,m), 3.58(3H,s), 4.10(2H,q,j=7.2Hz), 4.22(2H,q,j=7.2Hz), 4.60-4.87(1H,m), 6.29(1H,s), 7.24(2H,d,j=8.2Hz), 7.53(1H,d,j=7.6Hz), 7.72(2H,d,j=8.2Hz)

Example 13

Production of N-[4-[3-(2-amino-4-hydroxy-7-methyl-7H-pyrrolo[2,3-d]pyrimidine-5-yl)propyl]benzoyl]-L-glutamic acid :

The same hydrolysis reaction as in Example 5 was applied to the compound (1.54 g) of Example 12, thereby the subject compound (1.13 g) was obtained.

$^1$H-NMR ($Me_2SO$-$d_6$) δ : 1.81-2.18(4H,m), 2.23-2.41(2H,m), 2.53-2.81(4H,m), 3.58(3H,s), 4.26-4.57(1H,m), 6.01(2H,s), 6.42(1H,s), 7.26(2H,d,j=8Hz), 7.78(2H,d,j=8Hz), 8.47(1H,d,j=7.4Hz)

Example 14

Production of 4-[3-(2-amino-4-hydroxy-7-methyl-6,7-dihydro-5H-pyrrolo[2,3-d]pyrimidine-5-yl)propyl]benzoic acid :

Methyl 4-[3-(2-amino-3-isopropyloxymethyl-7-methyl-4(3H)-oxopyrrolo[2,3-d]pyrimidine-5-yl)-1-oxo-2-propenyl]benzoate (5.09 g) was dissolved in a mixed solution (240 ml) of methanol-tetrahydrofuran (1 :1), 1 N hydrochloric acid (24 ml) and 10 % Pd-C containing 50 % of water (2.6 g, produced by Engelhardt Co.) were added thereto, and the mixture was stirred vigorously for 48 hours under hydrogen atmosphere. The reaction mixture was filtered to remove the catalyst, and the filtrate was neutralized and concentrated under reduced pressure. The resultant residue was purified by silica gel chromatography (carrier ; 300 g, developing solvent ; chloroform-methanol = 1 :39--1 :19) to give methyl 4-[3-(2-amino-3-isopropyloxy-7-methyl-6,7-dihydro-4(3H)-oxopyrrolo[2,3-d]pyrimidine-5-yl)propyl]-benzoate of which the total amount was subjected to the same reaction removing isopropyloxymethyl group as in Example 1 and then hydrolyzed as in Example 2, thereby the subject

compound (1.02 g) was obtained.
$^1$H-NMR ($Me_2SO-d_6$) : 1.31-1.83(4H,m), 2.47-2.75(2H,m), 2.86-3.26(2H,m), 3.32-3.65(1H,m), 3.58(3H,s), 6.13(2H,s), 7.27(2H,d,j=8Hz), 7.79(2H,d,j=8Hz)

Example 15

Production of diethyl N-[4-[3-(2-mino-4-hydroxy-7-methyl-6,7-dihydro-5H-pyrrolo[2,3-d]pyrimidine-5-yl)propyl]benzoyl]-L-glutamate :

In the same manner as in Example 3, the subject compound (1.017 g) was obtained from the compound (0.985 g) of Example 14.
$^1$ H-NMR ($CDCl_3/CD_3OD$) : 1.19(3H,t,j=7.2Hz),1.27(3H,t,j=7.2Hz), 1.49-1.88(4H,m), 2.01-2.34(2H,m), 2.35-2.55(2H,m), 2.66(2H,brt,), 3.11-3.38(2H,m), 3.56(3H,s),3.52-3.78(1H,m), 4.08(2H,q,j=7.2Hz), 4.21(2H,q,j=7.2Hz), 4.57-4.89(1H,m), 5.70(2H,s), 7.26(2H,d,j=8Hz), 7.59(1H,d,j=7.5Hz), 7.75(2H,d,j=8Hz)

Example 16

Production of N-[4-[3-(2-amino-4-hydroxy-7-methyl-6,7-dihydro-5H-pyrrolo[2,3-d]pyrimidine-5-yl)propyl] benzoyl]-L-glutamic acid :

The same hydrolysis reaction as in Example 5 was applied to the compound (0.924 g) of Example 15, thereby the subject compound (0.716 g) was obtained.
$^1$H-NMR ($Me_2SO-d_6$) δ : 1.30-1.81(4H,m), 1.83-2.20(2H,m), 2.25-2.43(2H,m), 2.47-2.77(2H,m), 2.87-3.26(2H,m), 3.31-3.64(1H,m), 3.57(3H,s), 4.26-4.52(1H,m), 6.14(2H,s), 7.26(2H,d,j=8Hz), 7.78(2H,d,j=8Hz), 8.46(1H,d,j=7.4Hz)

Example 17

Production of methyl 4-[2-(4-amino-7-methylpyrrolo[2,3-d]pyrimidine-5-yl)ethyl]benzoate :

To a solution (25 ml) of the compound (90 mg) of Example 4 in methanol, 10 % Pd-C (45 mg) was added and the mixture was stirred at room temperature for 5 hours under hydrogen atmosphere. The catalyst was removed by filtration making use of sellaite, followed by removing the solvent in the filtrate by distillation under reduced pressure to give the subject compound (88 mg).
$^1$H-NMR ($CDCl_3$) δ : 3.07(4H,bs), 3.73(3H,s), 3.92(3H,s), 5.07(2H,m), 6.62(1H,s), 7.24,(2H,d,j=8.0Hz), 7.97(2H,d,j=8.0Hz), 8.29(1H,s)

Example 18

Production of diethyl N-[4-[2-(4-amino-7-methylpyrrolo[2,3-d]pyrimidine-5-yl)ethyl]benzoyl]-L-glutamate :

Under argon atmosphere, the compound (80 mg) of Example 17 was dissolved in a mixed solution (24 ml) of methanol-water-tetrahydrofuran (5 :5 :2), 1 N aqueous sodium hydroxide solution (0.76 ml) was added thereto, and the mixture was stirred at 40° C for 4 hours. To the resulting solution, 1 N hydrochloric acid (0.76 ml) was added, and then solvent was removed from the mixture by distillation under reduced pressure. The resultant residue was dried at 80° C under reduced pressure, thereby crude 4-[2-(4-amino-7-methylpyrrolo[2,3-d]pyrimidine-5-yl)ethyl]benzoic acid was obtained. Total amount of the crude compound and diethyl glutamate hydrochloride (92 mg) were dissolved in dried N,N-dimethylforamide (4 ml), a solution (2 ml) of diethyl cyanophosphate (50 mg) in dried N,N-dimethylformamide was added thereto at 0° C and the mixture was stirred for 15 minutes. Consecutively, at the same temperature, a solution (2 ml) of triethylamine (78 mg) in dried N,N-dimethylformamide was added dropwise thereto, and the mixture was stirred at 0° C for 30 minutes and then at room temperature for 14 hours. The resulting insoluble matters were removed by filtration, and solvent was removed from the filtrate by distillation under reduced pressure. The residue thus obtained was purified by silica gel chromatography (carrier : 15 g, developing solvent :

chloroform/ethanol containing 10 % of ammonia = 19/1) to give the subject compound (77 mg).
$^1$H-NMR ($CDCl_3$) δ : 1.23(3H,t,j=7.0Hz), 1.31(3H,t,j=7.0Hz), 2.08-2.53(4H,m), 3.07(4H,bs), 3.73(3H,s), 4.12(2H,q,j=7.0Hz), 4.25(2H,q,j=7.0Hz), 4.78-4.80(1H,m), 5.10(2H,m), 6.61(1H,s), 7.07(1H,d,j=7.6Hz), 7.25(2H,d,j=8.0Hz), 7.76(2H,d,j-8.0Hz), 8.29(1H,s)

Example 19

Production of N-[4-[2-(4-amino-7-methylpyrrolo[2,3-d]pyrimidine-5-yl)ethyl]benzoyl]-L-glutamic acid :

The product compound (69 mg) of Example 18 was dissolved in a mixed solution (10 ml) of tetrahydrofuran-water (3 :2), aqueous 1 N sodium hydroxide solution (0.45 ml) was added thereto, and the mixture was stirred at room temperature for 2 hours. Solvent was removed by distillation under reduced pressure from the reaction mixture and when its volume was reduced to about 5 ml, the mixture was filtered with Millipore filter, and then the filtrate was neutralized with 1 N hydrochloric acid (0.45 ml). The resulting precipitates were collected by filtration, washed with ice-water, methanol and ether in order, followed by drying at 60° C under reduced pressure to give the subject compound (22 mg).
$^1H$ -NMR ($Me_2SO-d_6$) δ : 1.90-2.15(2H,m), 2.32-2.41(2H,m), 2.91-3.00(2H,m), 3.06-3.16(2H,m), 3.61(3H,s), 4.37-4.45(1H,m), 6.51(2H,m), 6.87(1H,s), 7.36(2H,d,j=7.8Hz), 7.81(2H,d,j=7.8Hz), 8.04(1H,s), 8.48-8.52(1H,m)

Example 20

Production of N-[4-[3-(4-hydroxy-7-methylpyrrolo[2,3-d]pyrimidine-5-yl)propyl]benzoyl]-L-glutamic acid :
In the same manner as in Example 11, crude 4-[3-(4-hydroxy-7-methylpyrrolo[2,3-d]pyrimidine-5-yl)propyl]benzoic acid was obtained from methyl 4-[3-(4-hydroxy-7-methyl pyrrolo[2,3-d]pyrimidine-5-yl)-1-oxo-2-propenyl]benzoate. By subjecting the crude compound to the same reactions as in Examples 3 and 5, the subject compound was obtained.
$^1H$-NMR ($Me_2$-$d_6$+$D_2O$) δ : 1.70-2.20(4H,m), 2.25-2.38(2H,m), 2.61-2.77(4H,m), 3.58(3H,s), 4.35-4.44(1H,m), 6.81(1H,s), 7.32(2H,d,j=8Hz), 7.83(2H,d,j=8Hz), 8.43(1H,s)

Example 21

By manners according to Examples 1 to 20, there are obtained the following compounds ;
(1) N-[4-[2-(4-hydroxy-7-methylpyrrolo[2,3-d]pyrimidine-5-yl)ethyl]benzoyl]-L-glutamic acid,
(2) N-[4-[3-(4-amino-7-methylpyrrolo[2,3-d]pyrimidine-5-yl)propyl]benzoyl]-1-glutamic acid,

Example 22

By applying the same reduction reaction as in Example 9 to the respective compounds of Examples 19 to 21, there are obtained the following compounds ;
(1) N-[4-[2-(4-amino-7-methyl-6,7-dihydro-5H-pyrrolo[2,3-d]pyrimidine-5-yl)ethyl]benzoyl]-L-glutamic acid,
(2) N-[4-[3-(4-hydroxy-7-methyl-6,7-dihydro-5H-pyrrolo[2,3-d]pyrimidine-5-yl)propyl]benzoyl]-L-glutamic acid,
(3) N-[4-[2-(4-hydroxy-7-methyl-6,7-dihydro-5H-pyrrolo[2,3-d]pyrimidine-5-yl)ethyl]benzoyl]-L-glutamic acid,
(4) N-[4-[3-(4-amino-7-methyl-6,7-dihydro-5H-pyrrolo[2,3-d]pyrimidine-5-yl)propyl]benzoyl]-L-glutamic acid,
(5) N-[4-[3-(4-hydroxy-2-methyl-7-methyl-6,7-dihydro-5H-pyrrolo[2,3-d]pyrimidine-5-yl)propyl]benzoyl]-L -glutamic arid and
(6) N-[4-[2-(4-hydroxy-2-methyl-7-methyl-6,7-dihydro-5H-pyrrolo[2,3-d]pyrimidine-5-yl)ethyl]benzoyl]-L-g lutamic acid.

Example 23

Production of N-[4-[2-(2,4-diamino-7H-pyrrolo[2,3-d]pyrimidine-6-yl)ethyl]benzoyl]-L-glutamic acid :
Methyl 4-[2-(2,4-diamino-7H-pyrrolo[2,3-d]pyrimidine-6-yl)ethyl]benzoate (3.12 g) obtained from methyl 4-[3,3-dimethoxy-5-(2,4,6-triamino-pyrimidine-5-yl)benzyl]benzoate was sujected to the same reactions as in Examples 2, 3 and 5, thereby the subject compound (1.93 g) was obtained.
$^1H$-NMR ($Me_2SO-d_6$+$D_2O$) δ : 1.84-2.21(2H,m), 2.45(2H,t,j=8Hz), 2.98(4H,brs), 4.32-4.45(1H,m), 6.o6(1H,s), 7.33(2H,d,j=8Hz), 7.79(2H,d,j=8Hz)

Example 24

Production of N-[4-[2-(2,4-diamino-6,7-dihydro-5H-pyrrolo[2,3-d]pyrimidine-6-yl)ethyl]benzoyl]-L-glutamic acid :
By subjecting the compound (1.49 g) of Example 23 to the same reduction reaction as in Example 22 except

for employing plutinum oxide as a reduction catalyst instead of Pd-C, there was obtained the subject compound (0.5 g).
$^1$H-NMR($Me_2SO-d_6$) δ : 1.68-1.86(2H,m), 1.91-2.08(2H,m), 2.25-2.43(3H,m), 2.60-2.76(2H,m), 2/78-2.95(1H,s), 3.70-3.88(1H,m), 4.27-4.42(1H,m), 6.15(2H,brs), 6.29(2H,brs), 7.08(1H,brs), 7.33(2H,d,j=8Hz), 7.81(2H,d,j=8Hz), 8.35(1H,d,j=7.6Hz)

Example 25

Production of N-[4-[3-(2,4-diamino-7H-pyrrolo[2,3-d]pyrimidine-6-yl)propyl]benzoyl]-L-glutamic acid :
In the same manner as in Example 23, the subject compound (1.89 g) was obtained from methyl 4-[3-(2,4-diamino-7H-pyrrolo[2,3-d]pyrimidine-6-yl)propyl]benzoate (3.26 g).
$^1$H-NMR ($Me_2SO-d_6+D_2O$) δ : 1.75-2.22(4H,m), 2.36(2H,t,j=7Hz), 2.67(2H,t,j=7Hz), 2.70(2H,t,j=7Hz), 4.30-4.45(1H,m), 6.16(1H,s), 7.32(2H,d,j=8Hz), 7.81(2H,d,j=8Hz)

Example 26

Production of N-[4-[3-(2,4-diamino-6,7-dihydro-5H-pyrrolo[2,3-d]pyrimidine-6-yl)propyl]benzoyl]-L-glutamic acid :
In the same manner as in Example 24, the subject compound (0.726 g) was obtained from the compound (1.76 g) of Example 25.
$^1$H-NMR ($Me_2SO-d_6$) δ : 1.30-1.75(4H,m), 2.02(2H,dt,j=7.7Hz), 2.24-2.32(2H,m), 2.28(1H,dd,j=15.6Hz), 2.57-2.70(2H,m), 2.80(1H,dd,j=15.9Hz), 3.70-3.861H,m), 4.24-4.40(1H,m), 6.17(2H,brs), 6.34(2H,brs), 7.04(1H,brs), 7.29(2H,d,j=8Hz), 7.78(2H,d,j=8Hz), 8.33(1H,d,j=7Hz)

Example 27

By procedures similar to Examples 1 to 26, there are obtained the following compounds.
(1) N-[4-[2-(4-hydroxy-7H-pyrrolo[2,3-d]pyrimidine-6-yl)ethyl]benzoyl]-L-glutamic acid,
(2) N-[4-[3-(4-hydroxy-7H-pyrrolo[2,3-d]pyrimidine-6-yl)propyl]benzoyl]-L-glutamic acid,
(3) N-[4-[2-(4-hydroxy-6,7-dihydro-5H-pyrrolo[2,3-d]pyrimidine-6-yl)ethyl]benzoyl]-L-glutamic acid,
(4) N-[4-[3-(4-hydroxy-6,7-dihydro-5H-pyrrolo[2,3-d]pyrimidine-6-yl)propyl]benzoyl]-L-glutamic acid,
(5) N-[4-[2-(2-amino-4-hydroxy-6,7-dihydro-5H-pyrrolo[2,3-d]pyrimidine-6-yl)ethyl]benzoyl]-L-glutamic acid,
(6) N-[4-[3-(2-amino-4-hydroxy-6,7-dihydro-5H-pyrrolo[2,3-d]pyrimidine-6-yl)propyl]benzoyl]-L-glutamic acid,
(7) N-[4-[2-(2-amino-4-hydroxy-7-methyl-6,7-dihydro-5H-pyrrolo[2,3-d]pyrimidine-6-yl)ethyl]benzoyl]-L-g lutamic acid,
(8) N-[4-[3-(2-amino-4-hydroxy-7-methyl-6,7-dihydro-5H-pyrrolo[2,3-d]pyrimidine-6-yl)propyl]benzoyl]-L -glutamic acid,
(9) N-[5-[3-(2-amino-4-hydroxy-7H-pyrrolo[2,3-d]pyrimidine-6-yl)propyl]-5-thenoyl]-L-glutamic acid.

Example 28

Production of N-[4-[N-(2-amino-4-hydroxy-7H-pyrrolo[2,3-d]pyrimidine-6-ylmethyl)-N-methylamino]benzoyl]-L-glutamic acid :
6-(N,N-dimethylamino)methyl-4-hydroxy-2-octanoylamino-7H-pyrrolo[2,3-d]pyrimidine (2.34 g) was dissolved in a mixed solution (460 ml) of ethanol-tetrahydrofuran (1 :1), dimethyl sulfate (0.7 ml) was added thereto, and the mixture was stirred at room temperature for 28 hours. To the reaction mixture, diethyl N-(4-methylaminobenzoyl)-L-glutamate (5.72 g) and 28 % solution (1.35 g) of sodium methoxide in methanol was added and the mixture was stirred at room temperature for 66 hours. After adding 20 g of silica gel thereto, solvent was removed by distillation under reduced pressure. The resulting residue was purified by silica gel column chromatography (carrier ; 500 g, developing solvent ; chloroform : ethanol = 1 : 49) to give diethyl N-[4-[N-(2-octanoylamino-4-hydroxy-7H-pyrrolo[2,3-d]pyrimidine-6-ylmethyl)-N-methylamino]benzoyl]-L-gluta mate. To the total amount of this compound, the same hydrolysis reaction as in Example 5 was applied, thereby the subject compound (0.98 g) was obtained.
$^1$H-NMR ($Me_2SO-d_6+D_2O$) δ : 1.76-2.10(2H,m), 2.14-2.39(2H,m), 3.01(3H,s), 4.09-4.36(1H,m), 4.48(2H,s), 5.95(1H,s), 6.78(2H,d,j=8Hz), 7.65(2H,d,j=8Hz)

Example 29

Production of N-[5-[N-(2-amino-4-hydroxy-7H-pyrrolo[2,3-d]pyrimidine-6-ylmethyl)-N-methylamino]-2-thenoyl]-L-glutamic acid :

In the same manner as in Example 28, the subject compound (0.54 g) was obtained from 6-(N,N-dimethylamino)-methyl-4-hydroxy-2-octanoylamino-7H-pyrrolo[2,3-d]pyrimidine (1.67 g) and diethyl N-(5-methylamino-2-thenoyl)-L-glutamate (4.28 g).
$^1$H-NMR ($Me_2SO$-$d_6$+$D_2O$) δ : 1.81-2.12(2H,m), 2.15-2.38(2H,m), 3.12(3H,s), 4.13-4.35(1H,m), 4.51(2H,s), 5.97(1H,s), 6.02(1H,d,j=4Hz), 7.54(1H,d,j=4Hz)

Example 30

Production of N-[4-[N-(2-amino-4-hydroxy-7H-pyrrolo[2,3-d]pyrimidine-5-ylmethyl)-N-methylamino]benzoyl]-L-glutamic acid :

In the same manner as in Example 28, the subject compound (2.90 g) was obtained from N-[2-amino-4-hydroxy-7-methylpyrrolo[2,3-d]pyrimidine-5-yl]-N,N,N-trimethyl-ammonium methylsulfate (1.74 g) and diethyl N-[4-(N-methylamino)benzoyl]-L-glutamate (0.84 g).
$^1$H-NMR ($Me_2SO$-$d_6$+$D_2O$) δ : 1.08-2.07(2H,m), 2.11-2.19(2H,m), 3.05(3H,s), 3.61(3H,s), 4.10-4.18(1H,m), 4.63(2H,s), 6.77(1H,s), 7.29(2H,d,j=8Hz), 7.80(2H,d,j=8Hz)

Example 31

By applying procedure similar to Example 28 to respecive corresponding compounds, there are obtained the following compounds ;

(1) N-[5-[N-(2-amino-4-hydroxy-7-methylpyrrolo[2,3-d]pyrimidine-5-ylmethyl)-N-methylamino]-2-thenoyl] -L-glutamic acid,
(2) N-[4-[N-(2-amino-4-hydroxy-7-methylpyrrolo[2,3-d]pyrimidine-5-ylmethyl)N-propargylamino]benzoyl] -L-glutamic acid and
(3) N-[4-[N-(2-amino-4-hydroxy-7-methylpyrrolo[2,3-d]-pyrimidine-6-ylmethyl)-N-propargylamino]benzoyl] -L-glutamic acid.

Example 32

Production of N-[4-[2-(2,4-diaminothieno[2,3-d]pyrimidine-5-yl)ethyl]benzoyl]-L-glutamic acid :

Methyl 4-[2-(2,4-diamino-thieno[2,3-d]pyrimidine-5-yl)ethyl]benzoate (0.26 g) was subjected to the same reactions as in Examples 2, 3 and 5, thereby the subject compound (0.21 g) was obtained.
$^1$H-NMR ($Me_2SO$-$d_6$+$D_2O$) δ : 1.87-2.23(2H,m), 2.37(2H,t,j=7.4Hz), 2.96-3.08(2H,brt), 3.12-3.24(2H,brt), 4.34-4.47(1H,m), 6.54(1H,s), 7.33(2H,d,j=8.4Hz), 7.79(2H,d,j=8.4Hz)

Example 33

By applying procedures similar to Examples 1 to 32 to respective corresponding compounds the following compounds are obtained ;

(1) N-[4-[3-[2,4-diamino-thieno[2,3-d]pyrimidine-5-yl)propyl]benzoyl]-L-glutamic acid,
(2) N-[4-[3-(2-amino-4-hydroxythieno[2,3-d]pyrimidine-6-yl)propyl]benzoyl]-L-glutamic acid,
(3) N-[4-[3-(2-amino-4-hydroxyfuro[2,3-d]pyrimidine-6-yl)propyl]benzoyl]-L-glutamic acid,
(4) N-[4-[3-(2-amino-4-hydroxy-6,7-dihydro-5H-cyclopentapyrimidine-5-yl)propyl]benzoyl]-L-glutamic acid,
(5) N-[4-[3-(2-amino-4-hydroxy-6,7-dihydro-cyclopentapyrimidine-6-yl)propyl]benzoyl]-L-glutamic acid.

Example 34

Production of N-[4-[3-(4-hydroxy-2-methyl-7-methylpyrrolo[2.3-d]pyrimidine-5-yl)propyl]benzoyl]-L-glutamic acid :

Methyl 4-[3-(4-hydroxy-2-methyl-7-pyrrolo[2,3-d]pyrimidine-5-yl)propyl]benzoate (0.509 g) was subjected to the same reactions as in Examples 2, 3 and 5, thereby the subject compound (0.293 g) was obtained.
$^1$H-NMR ($Me_2SO$-$d_6$+$D_2O$) δ : 1.79-2.20(4H,m), 2.34(3H,s), 2.36(2H,t,j=7Hz), 2.73(2H,t,j=8Hz), 2.81(2H,t,j=8Hz), 3.62(3H,s), 4.35-4.46(1H,m), 6.78(1H,s), 7.31(2H,d,j=8Hz), 7.81(2H,d,j=8Hz).

Example 35

Production of N-[4-[2-(4-hydroxy-2-methyl-7-methylpyrrolo[2,3-d]pyrimidine-5-yl)ethyl]benzoyl]-L-glutamic acid :

Methyl 4-[3-(4-hydroxy-2-methyl-7-methylpyrrolo[2,3-d]pyrimidine-5-yl)ethyl]benzoate (0.749 g) was subjected to the same reactions as in Examples 2, 3 and 5, thereby the subject compound (0.486 g) was obtaind.
$^1$H-NMR ($Me_2$-$d_6$+$D_2O$) δ : 1.90-2.16(2H,m), 2.31(3H,s), 2.33(2H,t,j=7Hz), 2.75-3.03(4H,m), 3.58(3H,s), 4.32-4.47(1H,m), 6.76(1H,s), 7.30(2H,d,j=8Hz), 7.81(2H,d,j=8Hz).

Example 36

Production of N-[4-[2-(2-amino-4-hydroxy-7H-pyrrolo[2,3-d]pyrimidine-6-yl)ethyl]benzoyl-L-glutamic acid :

Methyl 4-[2-(2-amino-4-hydroxy-7H-pyrrolo[2,3-d]pyrimidine-6-yl)ethyl]benzoate (0.812 g) was subjected to the same reactions as in Examples 2, 3 and 5, thereby the subject compound (0.589 g) was obtained.
$^1$H-NMR ($Me_2SO$-$d_6$+$D_2O$) δ : 1.79-1.98(2H,m),7Hz), 2.34(2H,t,j=7Hz), 2.76-3.02(4H,m), 4.32-4.45(1H,m), 6.01(1H,s), 7.25(2H,d,j=8Hz), 7.74(2H,d,j=8Hz).

Example 37

Production of N-[4-[2-(2-amino-4-hydroxy-7H-pyrrolo[2,3-d]pyrimidine-6-yl)propyl]benzoyl]-L-glutamic acid :

Methyl 4-[2-(2-amino-4-hydroxy-7H-pyrrolo[2,3-d]pyrimidine-6-yl)propyl]benzoate (0.523 g) was subjected to the same reactins as in Examples 2, 3 and 5, thereby the subject compound (0.318 g) was obtained.
$^1$H-NMR ($Me_2SO$-$d_6$+$D_2O$) δ : 1.81-2.16(4H,m), 2.30(2H,t,j=7.4Hz), 2.52-2.85(4H,m), 4.38-4.53(1H,m), 6.04(1H,s), 7.27(2H,d,j=8Hz), 7.77(2H,d,j=8Hz).

Example 38

Production of N-[4-[2-(2-amino-4-hydroxy-7-methylpyrrolo[2,3-d]pyrimidine-6-yl)ethyl]benzoyl]-L-glutamic acid :

Methyl 4-[2-(2-amino-4-hydroxy-7methylpyrrolo[2,3-d]pyrimidine-6-yl)ethyl]benzoate (0.425 g) was subjected to the same reactions as in Examples 2, 3 and 5, thereby the subject compound (0.310 g) was obtained.
$^1$H-NMr ($Me_2SO$-$d_6$+$D_2O$) δ : 1.77-1.99(2H,m), 2.33(2H,t,j=7Hz), 2.72-3.03(4H,m), 3.60(3H,s), 4.33-4.47(1H,m), 6.00(1H,s), 7.26(2H,d,j=8Hz), 7.75(2H,d,j=8Hz).

Example 39

Production of N-[4-[2-(2-amino-4-hydroxy-7-methylpyrrolo[2,3-d]pyrimidine-6-yl)propyl]benzoyl]-L-glutamic acid :

Methyl 4-[2-(2-amino-4-hydroxy-7-methylpyrrolo[2,3-d]pyrimidine-6-yl)propyl]benzoate (0.341 g) was subjected to the same reactions as in Examples 2, 3 and 5, thereby the subject compound (0.282 g) was obtained.
$^1$H-NMR ($Me_2SO$-$d_6$+$D_2O$) δ : 1.80-2.14(4H,m), 2.31(2H,t,j=7.4Hz), 2.51-2.84(4H,m), 3.58(3H,s), 4.36-4.50(1H,m), 5.99(1H,s), 7.27(2H,d,j=8Hz), 7.78(2H,d,j=8Hz).

Exampe 40

Productin of N-[4-[2-(2-amino-4-hydroxy-7H-pyrrolo[2,3-d]pyrimidine-6-yl)ethyl]-2-thenoyl]-L-glutamic acid :

Methyl 4-[2-(2-amino-4-hydroxy-7H-pyrrolo[2,3-d]pyrimidine-6-yl)ethyl]thiophene-2-carboxylate (0.621 g) was subjected to the same reactions as in Examples 2, 3 and 5, thereby the subject compound (0.346 g) was obtained.
$^1$H-NMR ($Me_2SO$-$d_6$+$D_2O$) δ : 1.79-2.01(2H,m), 2.35(2H,t,j=7.4Hz), 2.74-3.01(4H,m), 4.37-4.51(1H,m), 5.98(1H,s), 6.81(1H,d,j=3.6Hz), 7.62(1H,d,j=3.5Hz).

Example 41

Production of N-[4-[2-(2-amino-4-hydroxythieno[2,3-d]pyrimidine-5-yl)ethyl]benzoyl]-L-glutamic acid :

Methyl 4-[2-(2-amino-4-hydroxythieno[2,3-d]pyrimidine-5-yl)ethyl]benzoate (0.824 g) was subjected to the

same reactions as in Examples 2, 3 and 5, thereby the subject compound (0.433 g) was obtained. $^1$H-NMR ($Me_2SO$-$d_6$) δ : 1.85-2.18(2H,m), 2.36(2H,t,j=7Hz), 2.88-3.13(4H,m), 4.34-4.47(1H,m), 6.55(1H,s), 7.32(2H,d,j=8Hz), 7.81(2H,d,j=8Hz).

Example 42

Production of N-[4-[2-(2-amino-4-hydroxythieno[2,3-d]pyrimidine-5-yl)propyl]benzoyl]-L-glutamic acid :

Methyl 4-[2-(2-amino-4-hydroxythieno[2,3-d]pyrimidine-5-yl)propyl]benzoate (0.721 g) was subjected to the same reactions as in Examples 2, 3 and 5, thereby the subject compound (0.356 g) was obatined. $^1$HNMR ($Me_2SO$-$d_6$+$D_2O$) δ : 1.75-2.12(4H,m), 2.35(2H,t,j=7Hz), 2.52-2.93(4H,m), 4.35-4.49(1H,m), 6.53(1H,s), 7.36(2H,d,j=8Hz), 7.88(2H,d,j=8Hz).

Example 43

Production of N-[4-[2-(2-amino-4-hydroxythieno[2,3-d]pyrimidine-6-yl)ethyl]benzoyl]-L-glutamic acid :

Methyl 4-[2-(2-amino-4-hydroxythieno[2,3-d]pyrimidine-6-yl)ethyl]benzoate (0.396 g) was subjected to the same reactions as in Examples 2, 3 and 5, thereby the subject compound (0.218 g) was obtained. $^1$H-NMR ($Me_2SO$-$d_6$+$D_2O$) δ : 1.81-2.05(2H,m), 2.32(2H,t,j=7Hz), 2.90-3.13(1H,m), 4.32-4.48(1H,m), 6.24(1H,s), 7.38(2H,d,j=8Hz), 7.89(2H,d,j=8Hz).

Example 44

Production of N-[4-[2-(2-amino-4-hydroxyfuro[2,3-d]pyrimidine-6-yl)ethyl]benzoyl]-L-glutamic acid :

Methyl 4-[2-(2-amino-4-hydroxyfuro[2,3-d]pyrimidine-6-yl)ethyl]benzoate (0.654 g) was subjected to the same reactions as in Examples 2, 3 and 5, thereby the subject compound (0.428 g) was obtained. $^1$H-NMR ($Me_2SO$-$d_6$+$D_2O$) δ : 1.84-2.08(2H,m), 2.34(2H,t,j=7Hz), 2.94-3.15(4H,m), 4.36-4.49(1H,m), 6.19(1H,s), 7.33(2H,d,j=8Hz), 7.90(2H,d,j=8Hz).

Example 45

Production of N-[4-[N-(2-amino-4-hydroxy-7-methylpyrrolo[2,3-d]pyrimidine-6-ylmethyl)-N-methylamino]benzoyl]-L-glutamic acid :

In the same manner as in Example 28, the subject compound (0.152 g) was obtained from N-[2-amino-4-hydroxy-7-methylpyrrolo[2,3-d]pyrimidine-6-yl]-N,N,N-trimethylammonium methylsulfate ((0.521 g) and diethyl N-[4-(N-methylamino)benzoyl]-L-glutamate (0.576 g). $^1$H-NMR ($Me_2SO$-$d_6$+$D_2O$) δ : 1.74-2.12(2H,m), 2.26(2H,t,j=7Hz), 3.02(3H,s), 3.57(3H,s), 4.12-4.38(1H,m), 4.46(2H,s), 5.98(1H,s), 6.77(2H,d,j=8Hz), 7.66(2H,d,j=8Hz).

Example 46

Productin of N-[4-[2-(2-amino-4-hydroxy-6,7-dihydro-5H-cyclopentapyrimidine-5-yl)ethyl]benzoyl]-L-glutamic acid :

Methyl 4-[2-(2-amino-4-hydroxy-6,7-dihydro-5H-cyclopentapyrimidine-5-yl)ethyl]benzoate 0.470 g) was subjected to the same reactions as in Examples 2, 3 and 5, thereby the subject compound (0.289 g) was obtained. $^1$H-NMR ($Me_2SO$-$d_6$+$D_2O$) δ : 1.38-2.13(8H,m), 2.20-2.55(4H,m), 2.58-2.79(3H,m), 4.23-4.39(1H,m), 7.31(2H,d,j=8Hz), 7.81(2H,d,j=8Hz).

Example 47

Production of N-[4-[2-(2-amino-4-hydroxy-6,7-dihydro-5H-cyclopentapyrimidine-6-yl)ethyl]benzoyl]-L-glutamic acid :

Methyl 4-[2-(2-amino-4-hydroxy-6,7-dihydro-5H-cyclopentapyrimidine-6-yl)ethyl]benzoate (0.392 g) was subjected to the same reactions as in Examples 2, 3 and 5, thereby the subject compound (0.241 g) was obtained. $^1$H-NMR ($Me_2SO$-$d_6$+$D_2O$) δ : 1.54-2.08(5H,m), 2.10-2.43(4H,m), 2.47-2.71(4H,m), 4.19-4.40(1H,m), 7.30(2H,d,J=8Hz), 7.79(2H,d,j=8Hz).

## Claims

1. A compound of the formula :

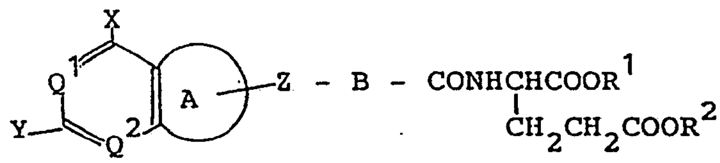

wherein the ring A is a 5-membered ring, provided that, in the case where the ring A is a pyrrole or pyrroline ring, (i) the N atom in the ring has no active proton or (ii) the ring bonds to Z at the -position of the ring ; B is a divalent cyclic or chain group which may be substituted ; one of $Q^1$ and $Q^2$ is N, with the other being N or CH ; Y is a hydrogen or halogen atom or a group which bonds to the bonding line with a carbon, nitrogen, oxygen or sulfur atom ; X is an amino, hydroxyl or mercapto group ;
Z is a straight-chain divalent group having a number of atoms of 2 to 5 composed of carbon atoms which each may be substituted, or of carbon atoms which each may be substituted and one hetero atom which may be substituted ;
$-COOR^1$ and $-COOR^2$ each is the same as or different from the other and represents a carboxyl group which may be esterified or its salt.

2. A compound according to Claim 1, wherein the ring A is N-substituted pyrrole or N-substituted pyrroline ring.

3. A compound according to Claim 1, wherein the ring A is N-methyl-pyrrole, N-methyl-pyrroline, N-methyl-pyrrolidine, thiophene, dihydrofuran or cyclopentene ring ; B is phenylene or thienylene group ; $Q^1$ and $Q^2$ each is N ; Y is H, $-CH_3$ or $-NH_2$ ; X is OH or $-NH_2$ ;

and Z is $-(CH_2)_n-$ (wherein n is 2 or 3) or $-CH_2\underset{CH_3}{\underset{|}{N}}-$ .

4. A compound according to Claim 1, which is diethyl N-[4-[2-(2-amino-7-benzyl-6-bromo-4-hydroxy-7H-pyrrolo[2,3-d]pyrimidine-5-yl)ethyl]benzolyl]-L-glutamic acid.

5. A compound according to Claim 1, which is diethyl N-[4-[2-(2-amino-7-benzyl-4-hydroxy-7H-pyrrolo[2,3-d]pyrimidine-5-yl)ethyl]benzoyl]-L-glutamic acid.

6. A compound according to Claim 1, which is N-[4-[2-(2-amino-7-benzyl-6-bromo-4-hydroxy-7H-pyrrolo[2,3-d]pyrimidine-5-yl)ethyl]benzoyl]-L-glutamic acid.

7. A compound according to Claim 1, which is N-[4-[2-(2-amino-7-benzyl-4-hydroxy-7H-pyrrolo[2,3-d]pyrimidine-5-yl)ethyl]benzoyl]-L-glutamic acid.

8. a compound according to Claim 1, which is dietyl N-[4-[2-(2-amino-4-hydroxy-7-methyl-7H-pyrrolo[2,3-d]pyrimidine-5-yl)ethyl]benzoyl]-L-glutamate.

9. A compound according to Claim 1, which is N-[4-[2-(2-amino-4-hydroxy-7-methyl-7H-pyrrolo[2,3-d]pyrimidine-5-yl)ethyl]benzoyl]-L-glutamic acid.

10. A compound according to Claim 1, which is diethyl N-[4-[2-(2-amino-4-hydroxy-2-methyl-6,7-dihydro-5H-pyrrolo[2,3-d]pyrimidine-5-yl)ethyl]benzoyl]-L-glutamate.

11. A compound according to Claim 1, which is N-[4-[2-(2-amino-4-hydroxy-7-methyl-6,7-dihydro-5H-pyrrolo[2,3-d]pyrimidine-5-yl)ethyl]benzoyl]-L-glutamate.

12. A compound according to Claim 1, which is diethyl N-[4-[3-(2-amino-4-hydroxy-7-methyl-7H-pyrrolo[2,3-d]pyrimidine-5-yl)propyl]benzoyl]-L-glutamate.

13. A compound according to Claim 1, which is N-[4-[3-(2-amino-4-hydroxy-7-methyl-7H-pyrrolo[2,3-d]pyrimidine-5-yl)propyl]benzoyl]-L-glutamic acid.

14. A compound according to Claim 1, which is diethyl N-[4-[3-(2-amino-4-hydroxy-7-methyl-6,7-dihydro-5H-pyrrolo[2,3-d]pyrimidine-5-yl)propyl]benzoyl]-L-glutamate.

15. A compound according to Claim 1, which is N-[4-[3-(2-amino-4-hydroxy-7-methyl-6,7-dihydro-5H-pyrrolo[2,3-d]pyrimidine-5-yl)propyl]benzoyl]-L-glutamic acid.

16. A compound according to Claim 1, which is diethyl N-[4-[2-(4-amino-7-methylpyrrolo[2,3-d]pyrimidine-5-yl)ethyl]benzoyl]-L-glutamate.

17. A compound according to Claim 1,. which is N-[4-[2-(4-amino-7-methylpyrrolo[2,3-d]pyrimidine-5-yl)ethyl]benzoyl]-L-glutamic acid.

18. A compound according to Claim 1, which is N-[4-[3-(4-hydroxy-7-methylpyrrolo[2,3-d]pyrimidine-5-yl)propyl]benzoyl]-L-glutamic acid.

19. A compound according to Claim 1, which is N-[4-[2-(4-hydroxy-7-methylpyrrolo[2,3-d]pyrimidine5-yl)ethyl]benzoyl]-L-glutamic acid.

20. A compound according to Claim 1, which is N-[4-[3-(4-amino-7-methypyrrolo[2,3-d]pyrimidine-5-yl)propyl]benzoyl]-L-glutamic acid.

21. A compound according to Claim 1, which is N-[4-[3-(4-hydroxy-2-methyl-7-methylpyrrolo[2,3-d]pyrimidine-5-yl)propyl]benzoyl]-L-glutamic acid.

22. A compound according to Claim 1, which is N-[4-[2-(4-hydroxy-2-methyl-7-methylpyrrolo[2,3-d]pyrimidine-5-yl)ethyl]benzoyl]-L-glutamic acid.

23. A compound according to Claim 1, which is N-[4-[2-(4-amino-7-methyl-6,7-dihydro-5H-pyrrolo[2,3-d]pyrimidine-5-yl)ethyl]benzoyl]-L-glutamic acid.

24. A compound according to Claim 1, which is N-[4-[3-(4-hydroxy-7-methyl-6,7-dihydro-5H-pyrrolo[2,3-d]pyrimidine-5-yl)propyl]benzoyl]-L-glutamic acid,

25. A compound according to Claim 1, which is N-[4-[2-(4-hydroxy-7-methyl-6,7-dihydro-5H-pyrrolo[2,3-d]pyrimidine-5-yl)ethyl]benzoyl]-L-glutamic acid.

26. A compound according to Claim 1, which is N-[4-[3-(4-amino-7-methyl-6,7-dihydro-5h-pyrrolo[2,3-d]pyrimidine-5-yl)propyl]benzoyl]-L-glutamic acid.

27. A compound according to Claim 1, which is N-[4-[3-(4-hydroxy-2-methyl-7-methyl-6,7-dihydro-5H-pyrrolo[2,3-d]pyrimidine-5-yl)propyl]benzoyl]-L-glutamic acid.

28. A compound according to Claim 1, which is N-[4-[2-(4-hydroxy-2-methyl-7-methyl-6,7-dihydro-5H-pyrrolo[2,3-d]pyrimidine-5-yl)ethyl]benzoyl]-L-glutamic acid.

29. A compound according to Claim 1, which is N-[4-[2-(2,4-diamino-7H-pyrrolo[2,3-d]pyrimidine-8-yl)ethyl]benzoyl]-L-glutamic acid.

30. A compound according to Claim 1, which is N-[4-[2-(2,4-diamino-6,7-dihydro-5H-pyrrolo[2,3-d]pyrimidine-6-yl)ethyl]benzoyl]-L-glutamic acid.

31. A compound according to Claim 1, which is N-[4-[3-(2,4-diamino-7H-pyrrolo[2,3-d]pyrimidine-5-yl)propyl]benzoyl]-L-glutamic acid :

32. A compound according to Claim 1, which is N-[4-[3-(2,4-diamino-6,7-dihydro-5H-pyrrolo[2,3-d]pyrimidine-6-yl)propyl]benzoyl]-L-glutamic acid :

33. A compound according to Claim 1, which is N-[4-[2-(4-hydroxy-7H-pyrrolo[2,3-d]pyrimidine-6-yl)ethyl]benzoyl]-L-glutamic acid.

34. A compound according to Claim 1, which is N-[4-[3-(4-hydroxy-7H-pyrrolo[2,3-d]pyrimidine-6-yl)propyl]benzoyl]-L-glutamic acid.

35. A compound according to Claim 1, which is N-[4-[2-(4-hydroxy-6,7-dihydro-5H-pyrrolo[2,3-d]pyrimidine-6-yl)ethyl]benzoyl]-L-glutamic acid.

36. A compound according to Claim 1, which is N-[4-[3-(4-hydroxy-6,7-dihydro-5H-pyrrolo[2,3-d]pyrimidine-6-yl)propyl]benzoyl]-L-glutamic acid.

37. A compound according to Claim 1, which is N-[4-[2-(2-amino-4-hydroxy-7H-pyrrolo[2,3-d]pyrimidine-6-yl)ethyl]benzoyl]-L-glutamic acid.

38. A compound according to Claim 1, which is N-[4-[3-(2-amino-4-hydroxy-7H-pyrrolo[2,3-d]pyrimidine-6-yl)ethyl]benzoyl]-L-glutamic acid.

39. A compound according to Claim 1, which is N-[4-[2-(2-amino-4-hydroxy-6,7-dihydro-5H-pyrrolo[2,3-d]pyrimidine-6-yl)ethyl]benzoyl]-L-glutamic acid.

40. A compound according to Claim 1, which is N-[4-[3-(2-amino-4-hydroxy-6,7-dihydro-5H-pyrrolo[2,3d]pyrimidine-6-yl)propyl]benzoyl]-L-glutamic acid.

41. A compound according to Claim 1, which is N-[4-[2-(2-amino-4-hydroxy-7-methylpyrrolo[2,3-d]pyrimidine-6-yl)ethyl]benzoyl]-L-glutamic acid.

42. A compound according to Claim 1, which is N-[4-[3-(2-amino-4-hydroxy-7-methylpyrrolo[2,3-d]pyrimidine-6-yl)propyl]benzoyl]-L-glutamic acid.

43. A compound according to Claim 1, which is N-[4-[2-(2-amino-4-hydroxy-7-methyl-6,7-dihydro-5H-pyrrolo[2,3-d]pyrimidine-6-yl)ethyl]benzoyl]-L-glutamic acid.

44. A compound according to Claim 1, which is N-[4-[3-(2-amino-4-hydroxy-7-methyl-6,7-dihydro-5H-pyrrolo[2,3-d]pyrimidine-6-yl)propyl]benzoyl]-L-glutamic acid.

45. A compound according to Claim 1, which is N-[5-[2-(2-amino-4-hydroxy-7H-pyrrolo[2,3-d]pyrimidine-6-yl)ethyl]-2-thenoyl]-L-glutamic acid.

46. A compound according to Claim 1, which is N-[5-[3-(2-amino-4-hydroxy-7H-pyrrolo[2,3-d]pyrimidine6-yl)propyl]-5-thenoyl]-L-glutamic acid.

47. A compound according to Claim 1, which is N-[4-[N-(2-amino-4-hydroxy-7H-pyrrolo[2,3-d]pyrimidine-6-ylmethyl)-N-methylamino]benzoyl]-L-glutamic acid.

48. A compound according to Claim 1, which is N-[5-[N-(2-amino-4-hydroxy-7H-pyrrolo[2,3-d]pyrimidine-6-ylmethyl)-N-methylamino]-2-thenoyl]-L-glutamic acid.

49. A compound according to Claim 1, which is N-[4-[N-(2-amino-4-hydroxy-7H-pyrrolo[2,3-d]pyrimidine-5-ylmethyl)-N-methylamino]benzoyl]-L-glutamic acid.

50. A compound according to Claim 1, which is N-[5-[N-(2-amino-4-hydroxy-7-methylpyriolo[2,3-d]pyrimidine-5-ylmethyl)-N-methylamino]-2-thenoyl]-L-glutamic acid.

51. A compound according to Claim 1, which is N-[4-[N-(2-amino-4-hydroxy-7-methylpyrrolo[2,3-d]pyrimidine-6-ylmethyl)-N-methylamino]benzoyl]-L-glutamic acid.

52. A compound according to Claim 1, which is N-[4-[N-(2-amino-4-hydroxy-7-methylpyrrolo[2,3-d]pyrimidine-5-ylmethyl)N-propargylamino]benzoyl]-L-glutamic acid.

53. A compound according to Claim 1, which is N-[4-[N-(2-amino-4-hydroxy-7-methylpyrrolo[2,3-d]pyrimidine-6-ylmethyl)-N-propargylamino]benzoyl]-L-glutamic acid.

54. A compound according to Claim 1, which is N-[4-[2-(2,4-diamino-thieno[2,3-d]pyrimidine-5-yl)ethyl]benzoyl]-L-glutamic acid.

55. A compound according to Claim 1, which is N-[4-[3-(2,4-diamino-thieno[2,3-d]pyrimidine-5-yl)propyl]benzoyl]-L-glutamic acid.

56. A compound according to Claim 1, which is N-[4-[2-(2-amino-4-hydroxythieno[2,3-d]pyrimidine-5-yl)ethyl]benzoyl]-L-glutamic acid.

57. A compound according to Claim 1, which is N-[4-[3-(2-amino-4-hydroxythieno[2,3-d]pyrimidine-5-yl)propyl]benzoyl]-L-glutamic acid.

58. A compound according to Claim 1, which is N-[4-[2-(2-amino-4-hydroxythieno[2,3-d]pyrimidine-6-yl)ethyl]benzoyl]-L-glutamic acid.

59. A compound according to Claim 1, which is N-[4-[3-(2-amino-4-hydroxythieno[2,3-d]pyrimidine-6-yl)propyl]benzoyl]-L-glutamic acid.

60. A compound according to Claim 1, which is N-[4-[2-(2-amino-4-hydroxyfuro[2,3-d]pyrimidine-6-yl)propyl]benzoyl]-L-glutamic acid.

61. A compound according to Claim 1, which is N-[4-[3-(2-amino-4-hydroxyfuro[2,3-d]pyrimidine-6-yl)propyl]benzoyl]-L-glutamic acid.

62. A compound according to Claim 1, which is N-[4-[2-(2-amino-4-hydroxy-6,7-dihydro-5H-cyclopentapyrimidine-5-yl)ethyl]benzoyl]-L-glutamic acid.

63. A compound according to Claim 1, which is N-[4-[3-(2-amino-4-hydroxy-6,7-dihydro-5H-cyclopentapyrimidine-5-yl)propyl]benzoyl]-L-glutamic acid.

64. A compound according to Claim 1, which is N-[4-[2-(2-amino-4-hydroxy-6,7-dihydro-5H-cyclopentapyrimidine-6-yl)ethyl]benzoyl]-L-glutamic acid.

65. A compound according to Claim 1, which is N-[4-[3-(2-amino-4-hydroxy-6,7-dihydro-cyclopentapyrimidine-6-yl)propyl]benzoyl]-L-glutamic acid.

66. A process for producing a compound according to Claim 1, which comprises reacting a compound represented by the formula :

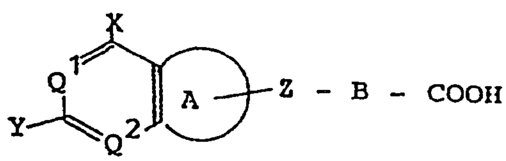

wherein the ring A is a 5-membered ring, provided that, in the case where the ring A is a pyrrole or pyrroline ring, (i) the N atom in the ring has no active proton or (ii) the ring bonds to Z at the -position of the ring ; B is a divalent cyclic or chain group which may be substituted ; one of $Q^1$ and $Q^2$ is N, with the other being N or CH ; Y is a hydrogen or halogen atom or a group which bonds to the bonding line with a carbon, nitrogen, oxygen or sulfur atom ; X is an amino, hydroxyl or mercapto group ;

Z is a straight-chain divalent group having a number of atoms of 2 to 5 composed of carbon atoms which each may be substituted, or of carbon atoms which each may be substituted and one hetero atom which may be substituted ;
or its rective derivative in respect to the carboxyl group with a compound as represented by the general formula :

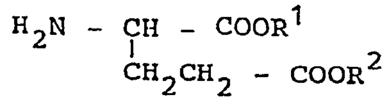

wherein -$COOR^1$ and -$COOR^2$ each is the same as or different from the other and represents a carboxyl group which may be esterified.

67. An antitumor composition which contains a compound according to Claim 1 or its salt.

68. A compound as represented by the general formula :

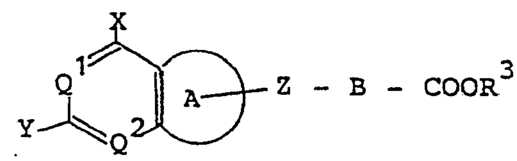

wherein the ring A is a 5-membered ring, provided that, in the case where the ring A is a pyrrole or pyrroline ring, (i) the N atom in the ring has no active proton or (ii) the ring bonds to Z at the $\alpha$ -position of the ring; B is a divalent cyclic or chain group which may be substituted ; one of $Q^1$ and $Q^2$ is N, with the other being N or CH ; Y is a hydrogen or halogen atom or a group which bonds to the bonding line with carbon, nitrogfen, oxygen or sulfur atom ; X is an amino, hydroxyl or mercapto group ;
Y is a hydrogen atom or a hydroxyl or amino group ; Z is a straight-chain divalent group having a number of atoms of 2 to 5 composed of carbon atoms which each may be substituted, or of carbon atoms which each may substituted and one hetero atom which may be substituted ; -$COOR^3$ is a carboxyl group which may be esterified, or its salt.

69. A compound according to Claim 68, which is methyl 4-[2-(2-amio-7-benzyl-6-bromo-4-hydroxy-7h-pyrrolo[2,3-d]pyrimidine5-yl]ethyl]benzoate.

70. A compound according to Claim 68, which is 4-[2-(2-amino-7-benzyl-6-bromo-4-hydroxy–7H-pyrrolo[2,3-d]pyrimidine-5-yl)ethyl]benzoic acid.

71. A compound according to Claim 68, which is 4-[3-(2-amino-4-hydroxy-7-methyl-7H-pyrrolo[2,3-d]pyrimidine-5-yl)propyl]benzoic acid.

72. A compound according to Claim 68, which is 4-[3-(2-amino-4-hydroxy-7-methyl-6,7-dihydro-5H-pyrrolo[2,3-d]pyrimidine-5-yl)propyl]benzoic acid.

73. A compound according to Claim 68, which is methyl 4-[2-(4-amino-7-methylpyrrolo[2,3-d]pyrimidine-5-yl)ethyl]benzoate.

74. A method for treating a subject suffering from tumor which comprises administering an effective amount of a compound of Claim 1 to the subject.

75. A use of a compound as claimed in Claim 1 or its salt in the manufacture of a medicament for the treatment of tumor.

76. A compound as claimed in Claim 1, wherein the ring A is a cyclopentadiene, cyclopentene, furan, dihydrofuran, thiophene, dihydrothiophene, thiophene-1-oxide, dihydrothiophene-1-oxide, thiophene-1, 1-dioxide, dihydrothiophone-1, 1-dioxide, N-subustituted pyrroline which may be substituted with one or two

members selected from the group consisting of a $C_{1-3}$ alkyl, $C_{2-4}$ alkenyl, $C_{2-3}$ alkynyl, cyclopropyl, halogen, $C_{1-4}$ alkanoyl, benzoyl, substituted benzoyl, cyano, carboxyl, carbamoyl, nitro, hydroxyl, hydroxymethyl, hydroxyethyl, methoxymethyl, ethoxymethyl, methoxyethyl, ethoxyethyl, $C_{1-3}$ alkoxy, mercapto, $C_{1-3}$ alkylthio, amino, substituted amino having 1 to 4 carbon atoms and $C_{1-4}$ alkanoylamino.

**77.** A compound as claimed in Claim 2 or 76, wherein the substituent of the N-substituted pyrrole and N-substituted pyrroline is selected from the group consisting of a $C_{1-3}$ alkyl, $C_{2-3}$ alkenyl, $C_{2-3}$ alkynyl, cyclopropyl, $C_{1-4}$ alkanoyl, benzoyl, p-chlorobenzoyl, p-methoxybenzoyl, 3,4,5-trimethoxybenzoyl, hydroxyethyl, methoxyethyl, ethoxyethyl, phenyl, p-chlorophenyl, p-methoxyphenyl, 3,4,5-trimethoxyphenyl, benzyl, p-chlorobenzyl, p-methoxybenzyl and diphenylmethyl.

**78.** A compound as claimed in Claim 76, wherein the substituted benzoyl is p-chlorobenzoyl, p-methoxybenzoyl or 3,4,5-trimethoxybenzoyl, and the substituted amino having 1 to 4 carbon atoms is methylamino, ethylamino, dimethylamino or diethylamino.

**79.** A compound as claimed in Claim 1, wherein X is a hydroxyl or amino group.

**80.** A compound as claimed in Claim 1, wherein X is a hydroxyl group.

**81.** A compound as claimed in Claim 1, wherein Y is hydrogen, or an amino or $C_{1-3}$ alkyl group.

**82.** A compound as claimed in Claim 1, wherein Y is an amino group.

**83.** A compound as claimed in Claim 1, wherein the ring A is a pyrrole, pyrroline, pyrrolidine, thiophene, dihydrofuran or cyclopentane which may be substituted with one or two selected from the group consisting of a halogen, benzyl ad $C_{1-3}$ alkyl.

**84.** A compound as claimed in Claim 1, wherein the ring A is a pyrrole or pyrroline group which is substituted with a halogen of/and benzyl.

**85.** A compound as claimed in Claim 1, wherein Z is an

ethylene, trimethylene or - 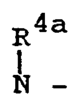 - wherein $R^{4a}$ is a $C_{1-3}$ alkyl group.

**86.** A compound as claimed in Claim 1, wherein Z is an ethylene group.

**87.** A compound as claimed in Claim 1, wherein Z is $-CH_2CH_2CH_2-$.

**88.** A compound as claimed in Claim 1, wherein B is a phenylene or thienylene group.

**89.** A compound as claimed in Claim 1, wherein B is

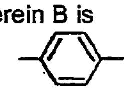 .

**90.** A compound as claimed in Claim 1, wherein $-COOR^1$ and $-COOR^2$ each represents a carboxyl group which may be esterified with a $C_{1-5}$ alkyl group.

**91.** A compound as claimed in Claim 1, wherein the ring A is a pyrrole or pyrroline ring bonding to Z at the α-position.

**92.** A compound as claimed in Claim 1, wherein X and Y each is an amino group and Z is $-CH_2CH_2CH_2-$.